# EUROPÄISCHE PATENTSCHRIFT

(11) **EP 1 458 363 B1**
(45) Veröffentlichungstag und Bekanntmachung des Hinweises auf die Patenterteilung: **16.01.2013**
(21) Anmeldenummer: 02792980.1
(22) Anmeldetag: 12.12.2002
(51) Int. Cl.: A61K 9/16

(54) **PRÄFORMULIERUNG FÜR DIE TABLETTIERUNG VON NATÜRLICHEN GEMISCHEN KONJUGIERTER OESTROGENE**
PRE-FORMULATION FOR THE TABLETTING OF NATURAL MIXTURES OF CONJUGATED ESTROGENS
PREFORMULATION POUR LA MISE EN COMPRIMES DE MELANGES NATURELS D'OESTROGENES CONJUGUES

(30) Priorität: 14.12.2001 EP 01129840
(43) Veröffentlichungstag der Anmeldung: 22.09.2004
(73) Patentinhaber: Abbott Products GmbH, 30173 Hannover (DE)
(72) Erfinder: THUMBECK, Bernd, 31171 Nordstemmen (DE); BONNACKER, Ingo, 31171 Nordstemmen (DE); LERCH, Martina, 30855 Langenhagen (DE)
(74) Vertreter: Hart-Davis, Jason
(86) Internationale Anmeldenummer: PCT/EP2002/014104
(87) Internationale Veröffentlichungsnummer: WO 2003/051337

(56) Entgegenhaltungen:
- EP-A- 0 322 020
- WO-A-97/04752
- US-A- 3 487 152
- "Chapter 2" In: Wolfgang A. Ritschel, Annette Bauer-Brandl: "Die Tablette", 2002
- 'Pharmazeutische Pellets - Herstellung, Eigenschaften und Anwendung', ISBN 3-9808514-2-7 Artikel 'Mikrokristalline Cellulose'
- "Celluloseether" In: "Roempp", 2005, Georg Thieme Verlag
- "Cellulose-Derivate" In: 2005, Georg Thieme Verlag
- 'Pharmaceutical significance of cellulose: A review' Bd. 2, Nr. 11, 2008, Seiten 758 - 778
- SIEPMANN AND PEPPAS: 'Modeling of drug release from delivery systems based on hydroxypropyl methylcelulose (HPMC)' Bd. 48, 2001, Seiten 139 - 157
- FIEDLER: 'Encyclopedia of Excipients', 2002 Seiten 380-381 - 855-856
- PARMENTIER: 'Auxiliary Agents for Direct Tabletting - A Survey' PHARMA INTERNATONAL Seiten 18 - 22

## Beschreibung

Die vorliegende Erfindung betrifft pharmazeutische Präformulierungen in Form eines Trockenextraktes von natürlichen Gemischen konjugierter equiner Oestrogene, insbesondere von Gemischen aus dem Harn trächtiger Stuten gewonnener, konjugierter Oestrogene. Hierdurch werden Trockenextrakte als für die Herstellung von festen galenischen Formen, z.B. für die Tablettierung, geeignete Präformulierungen von diesen natürlichen Gemischen konjugierter Oestrogene bereitgestellt. Die Erfindung betrifft weiterhin ein Verfahren zur Herstellung dieser Präformulierungen in Form eines Trockenextraktes.

Oestrogene werden in der Medizin zur Hormonsubstitutionstherapie eingesetzt. Insbesondere werden Oestrogen-Gemische zur Behandlung und Prophylaxe der bei Frauen auftretenden Beschwerden der Wechseljahre nach natürlicher oder artifizieller Menopause eingesetzt. Hierbei haben sich natürliche Gemische konjugierter equiner Oestrogene, wie sie im Harn trächtiger Stuten vorliegen, als besonders wirksam und gut verträglich erwiesen.

Der gelöste Feststoffgehalt im Harn trächtiger Stuten (= pregnant mares' urine, im folgenden abgekürzt als "PMU") kann natürlicherweise in weiten Bereichen schwanken und im allgemeinen in einem Bereich von 40 bis 90 g Trockensubstanz pro Liter liegen. Neben Harnstoff und sonstigen üblichen Harninhaltsstoffen sind im Feststoffgehalt des PMU phenolische Bestandteile, z.B. Kresole und das als HPMF bekannte Dihydro-3,4-bis[(3-hydroxyphenyl)methyl]-2(3H)-furanon, enthalten. Das im PMU enthaltene natürliche Gemisch von Oestrogenen liegt weitgehend in konjugierter Form, z.B. als Schwefelsäurehalbester-Natriumsalz (im folgenden abgekürzt als "Sulfatsalz"), vor. Der Gehalt an konjugierten Oestrogenen (= conjugated estrogen, im folgenden abgekürzt als "CE") kann berechnet als Oestrogensulfatsalz und bezogen auf Trockensubstanz zwischen 0,3 und 1 Gew.-% betragen.

Durch Abtrennung der unerwünschten Begleitstoffe, wie Harnstoff und insbesondere Kresole und HPMF, werden aus dem PMU üblicherweise Extrakte gewonnen, welche die konjugierten Oestrogene aus dem Harn trächtiger Stuten (PMU) in gelöster Form enthalten (Lösungsextrakte). Neuere Methoden gewinnen hierbei natürliche Gemische dieser konjugierten Oestrogene (CE) durch Festphasenextraktion des Gemisches konjugierter Oestrogene aus dem Harn trächtiger Stuten z.B. an RP-Kieselgel (WO 98/08525) oder an semipolaren, insbesondere an nichtionischen semipolaren, polymeren Adsorberharzen (WO 98/08526). Mit diesen Methoden können die unerwünschten Begleitstoffe in effektiver und effizienter Weise aus dem PMU abgetrennt und wässrige Lösungsextrakte der CE von guter Qualität gewonnen werden. Die Konzentration der CE im Lösungsextrakt unterliegt jedoch gewissen, unvermeidlichen Schwankungen, da das für die Gewinnung der CE eingesetzte PMU als ein natürliches Ausgangsmaterial per se natürlichen Qualitätsschwankungen aufgrund Herkunft, Lagerung, Transport und eventueller Vorverarbeitung etc. unterliegt.

Bei der Herstellung von pharmazeutischen Zubereitungen von natürlichen Gemischen konjugierter equiner Oestrogene aus CE-enthaltenden Lösungsextrakten muss eine gleichbleibende Qualität und Dosierungsstärke der Zubereitung sichergestellt werden. Die in Abhängigkeit von Ausbeute und Qualität des Ausgangsmaterials auftretenden natürlichen Schwankungen des Gehaltes konjugierter equiner Oestrogene in den für die Herstellung pharmazeutischer Zubereitungen verwendeten Lösungsextrakte müssen daher durch geeignete Maßnahmen ausgeglichen werden, um für die weitere galenische Verarbeitung ein Material konstanter Qualität und definierter Spezifikation bereitzustellen.

Es besteht daher ein dringender Bedarf an geeigneten verbesserten Verfahren zur möglichst produktschonenden Überführung der durch Aufarbeitung von PMU gewonnenen CE-enthaltenden wässrigen Lösungsextrakte in eine feste galenische Präformulierung, die das natürliche Gemisch konjugierter equiner Oestrogene als Wirkkomponente in definierter Form und Konzentration sowie in homogener Verteilung enthält und als Wirkstoff-haltiger fester pharmazeutischer Rohstoff ("Trockenextrakt") dann in einfacher Weise für die weitere galenischen Verarbeitung zu festen Formen, wie beispielsweise zur Tablettierung oder Direkttablettierung, eingesetzt werden kann. Das natürliche Gemisch konjugierter equiner Oestrogene muss somit in einer Form vorliegen, welche die chemische Stabilität der Hormone, d.h. der in den Gemischen enthaltenen konjugierten Oestrogene, gewährleistet und welche die Verarbeitung dieser Hormone in eine feste galenische Form, z.B. in eine Tablette, ermöglicht.

In der internationalen Anmeldung WO 97/04752 wird ein Verfahren zur Herstellung einer konjugierte Östrogene enthaltenden Matrixtablette mit kontrollierter Wirkstofffreisetzung beschrieben, in dem konjugierte Östrogene in definierter Ausgangsmenge in einem hydroalkoholischen Lösungsmittel gelöst und dann auf organischer Trägerstoffe aufgesprüht wird, wobei diese Trägerstoffe eine Kombination gelbildender, das Freisetzungsverhalten beeinflussende organische Polymere wie Cellulose-Derivate, beispielsweise Hydroxypropylmethylcellulose, und nicht gelbildenden organischen Trägerstoffen wie Laktose sind. Nach Aufsprühen einer definierten Menge an konjugierten Östrogenen wird getrocknet und anschließend mit anderen Tablettierhilfsstoffen granuliert und zu Tabletten verpresst.

Aufgabe der vorliegenden Erfindung ist somit die Bereitstellung eines festen, freifließenden pharmazeutischen Rohstoffes (Trockenextrakt) als Ausgangsmaterial für die galenische Zubereitung von CE-enthaltenden Pharmazeutika in fester Form, beispielsweise durch Tablettierung oder Direkttablettierung, wobei der als Ausgangsmateriäl für die galenische Verarbeitung bereitgestellte Rohstoff das aus dem Harn trächtiger Stuten gewonnene natürliche Gemisch konjugierter equiner Oestrogene als Wirkkomponente in definierter Form, homogener Verteilung sowie einen definierten, standardisierten Wirkstoffgehalt auf einem pharmazeutischen Trägerstoff enthält.

Es wurde nun überraschenderweise gefunden, dass durch Aufarbeitung von PMU gewonnene wässrige Lösungsextrakte, die ein Gemisch natürlicher konjugierter equiner Oestrogene (CE) enthalten, in einfacher Weise als Wirkstoffkomponente auf einen, in einer Wirbelschicht fluidisierten festen pharmazeutischen Trägerstoff in homogener Verteilung und mit definierter, standardisierter Konzentration aufgebracht werden können, wobei Wirkstoff-beschichtete Partikel definierter Form entstehen und der Wirkstoff, d.h. das natürliche Gemisch konjugierter equiner Oestrogene, sehr stabil in einer festen Form gebunden vorliegt. Diese pharmazeutische Präformulierung kann als festes, freifließendes Pulver und/oder granulatförmiges Trockenextrakt von hoher Qualität bequem galenisch, insbesondere durch Tablettierung oder Direkttablettierung, zu festen pharmazeutischen Zubereitungen weiterverarbeitet werden.

Die vorliegende Erfindung betrifft daher eine pharmazeutische Präformulierung in Form eines festen, freifließenden Trockenextraktes für die Tablettierung, gekennzeichnet durch
(a) einen pro Menge Trägermaterial definierten, (bezogen auf die Haupthormon-Komponenten) standardisierten Wirkstoffgehalt eines Gemisches natürlicher konjugierter equiner Oestrogene, wobei
(b) der Wirkstoffgehalt durch Aufsprühen aus einer wässrigen Lösung auf einen pulver- und/oder granulatförmigen pharmazeutischen Trägerstoff aus der Gruppe mikrokristalliner Cellulosen oder einem Gemisch von mikrokristalliner Cellulose mit Lactose und Trocknung aufgebracht ist.

Konjugierte equine Oestrogene sind ein Gemisch verschiedener konjugierter Formen von Oestrogenen, die aus dem Harn trächtiger Stuten erhalten werden. Die zwei prinzipiellen Hauptkomponenten sind Natriumestronsulfat und Natriumequilinsulfat. Eine dritte wesentliche Komponente ist das 17-α-Dihydroequilinsulfat. Daneben sind auch noch Natrium-17-α-Estradiolsulfat und Natrium-17-β-Dihydroequilinsulfat von Bedeutung. Konjugierte Oestrogene (CE) enthalten üblicherweise 52,5 bis 61,5 Gew.-% Natriumestronsulfat, 22,5 bis 30,5 Gew.-% Natriumequilinsulfat, 13,5 bis 19,5 Gew.-% Natrium-17-α-Dihydroequilinsulfat, 2,5 bis 9,5 Gew.-% Natrium-17-α-Estra-diolsulfat und 0,5 bis 4 Gew.-% Natrium-17-β-Dihydroequilin-sulfat. Der Gesamtanteil an Natriumestronsulfat und Natriumequilinsulfat liegt üblicherweise im Bereich von 79,5 bis 88 Gew.-%. Der Gesamtgehalt an freien Oestrogenen wie Estron, Equilin und 17-α-Dihydroequilin beträgt üblicherweise nicht mehr als 1,3 Gew.-%. Die vorstehenden Prozent-Angaben beziehen sich auf den sogenannten "Labeled Cöntent", wie dieser gemäß European Pharmacopoeia 2001 oder analog der USP (United States Pharmacopoeia) über gaschromatographische Profile, im Vergleich zu Referenzlösungen üblicherweise ermittelt und berechnet werden kann.

Der Wirkstoffgehalt der im Gemisch natürlicher konjugierter equiner Oestrogene enthaltenen Hormone wird üblicherweise auf die Haupthormon-Komponenten standardisiert, wobei in der Regel auf die Summe der drei Hauptkomponenten Estron, Equilin und 17-α-Dihydroequilin und gelegentlich aber auch auf die Summe aus diesen drei Hauptkomponenten und zusätzlich 17-α-Estradiol und 17-β-Dihydroequilin abgestellt wird (jeweils konjugierte und freie Hormone).

In zweckmäßigen Ausgestaltungen der vorliegenden Erfindung zeichnet sich die pharmazeutische Präformulierung dadurch aus, dass der Wirkstoffgehalt berechnet als Trockensubstanz (TS) eines das Gemisch natürlicher konjugierter equiner Oestrogene enthaltenden Extraktes aus dem Harn trächtiger Stuten (Gesamthormongehalt einschließlich der freien Oestrogene und sonstiger Feststoffe) bezogen auf die Menge des pharmazeutischen Trägerstoffes in der Präformulierung im Bereich von 0,25 bis 0,70 g TS/g Trägerstoff, vorzugsweise im Bereich von 0,28 bis 0,64 g TS/g Trägerstoff, liegt.

Wird der Wirkstoffgehalt (Gesamthormongehalt einschließlich der freien Oestrogene) der pharmazeutischen Präformulierung als Gemisch natürlicher equiner konjugierter Oestrogene (CE) bezogen auf die Menge des pharmazeutischen Trägerstoffes in der Präformulierung berechnet, so liegt der Wirkstoffgehalt im Bereich von 35 bis 100 mg CE/g Trägerstoff, vorzugsweise im Bereich von 43 bis 90 mg CE/g Trägerstoff.

Nach Trocknung der durch Aufsprühen des CE-Wirkstoffgehaltes aus einer wässrigen Lösung auf den pulver- und/oder granulatförmigen pharmazeutischen Trägerstoff aus der Gruppe mikrokristalliner Cellulosen oder auf ein Gemisch von wenigstens einer dieser mikrokristallinen Cellulosen mit Lactose erhaltenen pharmazeutischen Präformulierung, kann diese herstellungsbedingt noch einen geringen Anteil an Restfeuchte aufweisen. Üblicherweise liegt der Restfeuchteanteil hierbei im Rahmen der bei den angewendeten Trocknungsverfahren üblichen Maximalwerte. So beträgt die Restfeuchte in der pharmazeutischen Präformulierung insbesondere maximal etwa 3,0 Gew.-%, vorzugsweise maximal etwa 1,0 Gew.-%, bezogen auf die gesamte Präformulierung als 100 Gew.-% (Summe aus dem als Trockensubstanz berechneten Wirkstoffgehalt, dem pharmazeutischen Trägerstoff und Berücksichtigung des Anteils an Restfeuchte).

Wird der Wirkstoffgehalt der erfindungsgemäßen pharmazeutischen Präformulierung als Gesamthormongehalt (Summe aller konjugierten und freien Hormone) berechnet, so liegt der Wirkstoffgehalt im Bereich von etwa 35 bis 100 mg pro 1 g des pharmazeutischen Trägerstoffes, vorzugsweise im Bereich von etwa 43 bis 90 mg pro 1 g des pharmazeutischen Trägerstoffes.

Zweckmäßige Ausführungsformen der erfindungsgemäßen pharmazeutischen Präformulierung, zeichnen sich dadurch aus, im Wirkstoffgehalt die konjugierten Hormone(jeweils als Natriumsalz des Sulfatesters), insbesondere die konjugierten Haupthormone, in folgenden Anteilen enthalten sind: 52,5 bis 61,5 % Estron, 22,5 bis 30,5 % Equilin, 13,5 bis 19,5 % 17-µ-Dihydroequilin, 2,5 bis 9,5 % Estradiol, 0,5 bis 4,0 % 17-µ-Dihydroequilin.

Weiterhin liegt in vorteilhaften Varianten der erfindungsgemäßen pharmazeutischen Präformulierung der Summenanteil an freien Hormonen in der Präformulierung im Bereich von maximal etwa 2 bis 3 mg pro 1 g des pharmazeutischen Trägerstoffes. Vorzugsweise liegt der Anteil an freien Hormonen im Wirkstoffgehalt der Präformulierung bezogen auf den Gesamtgehalt an Hormonen (Summe aller konjugierten und freien Hormone) unter 5 Gew.-%. Je nach Aufarbeitung des zur Herstellung der erfindungsgemäßen pharmazeutischen Präformulierung eingesetzten hormonhaltigen wässrigen Lösungsextraktes kann der auf den Gesamthormongehalt bezogene Anteil an freien Hormonen auch deutlich darunter liegen, z.B. unter 2 Gew.-%.

Es zeigte sich überraschenderweise, dass durch Aufsprühen eines aus PMU gewonnenen CE-Lösungsextraktes durch die Wirbelschichttechnik auf bestimmte pharmazeutische Tägerstoffe, nämlich mikrokristalline Cellulosen oder Gemische dieser mikrokristallinen Cellulosen mit Lactose, die konjugierten Hormone auf diese Trägerstoffe homogen aufgetragen werden können und dass sich das hierdurch erhaltene feste, freifließende Trockenextrakt vorteilhaft eignet, um feste galenische Formen, wie z.B. Tabletten, herzustellen. Insbesondere können die erfindungsgemäßen pharmazeutischen Präformulierungen in Form des Trockenextraktes homogen in eine Tablette, vorzugsweise in eine Matrix-Tablette, verteilt und verpresst werden, wobei erwünschte Freisetzungsprofile erzielt werden können. Überraschenderweise zeigte sich hierbei auch, dass durch die Wahl des pharmazeutischen Trägerstoffes in Abhängigkeit der Wasserlöslichkeit des Trägerstoffes oder TrägerstoffGemisches die Freisetzungsgeschwindigkeit von in einer Matrix-Tablette verpresst vorliegenden konjugierten Hormonen vorteilhaft beeinflusst werden kann. Hierbei beeinflussen insbesondere die Art und Zusammensetzung des pharmazeutischen Trägerstoffes bzw. Trägerstoffgemisches, also die Art und die Eigenschaften mikrokristalliner Zellulose und Lactose, die Partikelgrösse und die Porosität des wirkstoffgranulates und die Partikelgrössenverteilung vorteilhaft die Qualität der Verpressbarkeit der erfindungsgemäß erhaltenen pharmazeutischen Präformulierung und in der Folge das Freisetzungsprofil der konjugierten Hormone aus einer mittels dieser pharmazeutischen Präformulierung hergestellten Matrix-Tablette. Weiterhin können , obgleichnicht Teil der beanspruchten Erfindung, neben den vorstehend genannten erfindungsgemäß ausgewählten pharmazeutischen Trägerstoffen oder Trägerstoff-Gemischen geringe Mengen weiterer üblicher Tablettier-Hilfstoffe bzw. Stabilisatoren in der erfindungsgemäßen pharmazeutischen Präformulierung in geringer Menge vorliegen, wodurch eine weitere Beeinflussung des Freisetzungsprofils der Hormone sowie deren Stabilität in der pharmazeutischen Präformulierung oder daraus hergestellten festen pharmazeutischen Zubereitungen wie Tabletten, insbesondere MatrixTabletten, ermöglicht wird. Solche Tablettier-Hilfstoffe sind z.B. Füllstoffe, Sprengmittel, Zerfallsförderer- oder - beschleuniger, Trockenbindemittel, Trockenmittel oder Adsorptionsmittel, Gleitmittel (z.B. Fließregulierungs-, Schmier- oder Formtrennmittel). Diese beispielhaft genannten Tablettier-Hilfsstoffe, oder auch weitere dem Fachmann geläufige und üblicherweise in der Tablettenherstellung verwendete Hilfsstoffe, können zu den erfindungsgemäßen Präformulierungen maximal in solchen Mengen zugemischt werden, in denen sie auch in der fertigen Matrixtablette vorliegen sollen.

Die erfolgreiche Verwendbarkeit der erfindungsgemäßen Präformulierung zur Herstellung von festen galenischen Formen natürlicher Gemische konjugierter equiner Oestrogen, insbesondere z.B. von Tabletten oder vorzugsweise Matrix-Tabletten, stellt einen wesentlichen Teilschritt bei der Herstellung der eigentlichen festen galenischen Form für die therapeutische bzw. prophylaktische Verabreichung an Patienten dar und beruht neben anderen Faktoren auch auf der Art der erfindungsgemäß ausgewählten pulver- und/oder granulatförmigen pharmazeutischen Trägerstoffe, nämlich pharmazeutische Trägerstoffe aus der Gruppe mikrokristalliner Cellulosen und die fakultativ im Gemisch mit mikrokristalliner Cellulose eingesetzte Lactose. Ist der pharmazeutische Trägerstoff in der erfindungsgemäßen pharmazeutischen Präformulierung eine mikrokristalline Cellulose, so kann diese eine einzelne Type mikrokristalliner Cellulose sein oder auch ein Gemisch verschiedener Typen mikrokristalliner Cellulosen. Eine andere Variante der Erfindung enthält Gemische von mikrokristalliner Cellulose mit Lactose, die jeweils in pulver- und/oder granulatförmiger Form vorliegen. In der Variante der erfindungsgemäßen Präformulierungen, in der Gemische aus einer mikrokristallinen Cellulose mit Lactose als Trägerstoff vorliegen, kann deren Mischungsverhältnis in weiten Bereichen variiert werden, wobei jedoch zweckmäßigerweise darauf zu achten ist, dass die Menge der mikrokristalliner Cellulose nicht unter 60 Gew.-%, vorzugsweise nicht unter 80 Gew.-%, und die Menge der Lactose nicht über 40 Gew.-%, vorzugsweise nicht über 20 Gew.-%, liegen sollte. Zweckmäßige Mischungsverhältnisse von mikrokristalliner Cellulose zu Lactose sind gegeben, sofern das Gewichtsverhältnis von mikrokristalliner Cellulose zu Lactose im Bereich von 8:2 bis 6:4 liegt, vorzugsweise im Bereich von 7,5:2,5 bis 6,5:3,5. In einer beispielhaften Ausgestaltung der erfindungsgemäßen Präformulierung beträgt das Mischungsverhältnis von mikrokristalliner Cellulose zu Lactose etwa 7:3 als Gewichtsverhältnis.

Mikrokristalline Cellulosen sind als pharmazeutischer Grundstoff in verschiedenen Ausführungen im Handel erhältlich, z.B. als Avicel^{®} (z.B. der Firma Lehmann & Voss & Co., Hamburg, Deutschland), insbesondere als Avicel^{®}-Typen PH 101, PH 102, PH 102 SCG oder PH 103. Die als Avicel^{®} im Handel erhältlichen mikrokristallinen Cellulosen für pharmazeutische Zwecke weisen üblicherweise z.B. folgende allgemeine Spezifikation auf: Wassergehalt unter 5 Gew.-% (Type PH 103: unter 3 Gew.-%); Asche unter 10; Brechungsindex 1,55; pH (Dispersion) 5,5 bis 7,0; mittlere Korngrößen für

| Type | PH 101 | PH 102 | PH 102 SCG | PH 103 |
|---|---|---|---|---|
| | 50 µm | 100 µm | 130 µm | 50 µm; |

und ein Teilchengrößenverteilung von:

| Type | PH 101 | PH 102 | PH 102 SCG | PH 103 |
|---|---|---|---|---|
| 250 µm | < 1 % | < 8 % | < 8 % | < 1 % |
| 150 µm | | | > 23 % | |
| 75 µm | < 30 % | > 45 % | > 63 % | < 30 % |

Eine weitere, erfindungsgemäß verwendbare, im Handel erhältliche mikrokristalline Cellulose für pharmazeutische Zwecke wird unter dem Handelsnamen Vivapur^{®}, z.B als Type Vivapur^{®} 101 oder Vivapur^{®} 12, vertrieben (z.B. von der Firma J. Rettenmaier & Söhne GmbH + Co, Rosenberg, Deutschland). Vivapur^{®} 101 weist üblicherweise z.B. folgende allgemeine Spezifikation auf: Trocknungsverlust maximal 6 Gew.-%; Polymerisationsgrad (Identität) < 350; Schüttdichte 0,26 bis 0,32 g/ml; Korngrößenverteilung: d₁₀: < 30 µm, d₅₀: 40 bis 70 µm, d₉₀: > 80 µm; Siebanalyse (Rückstand auf dem Luftstrahlsieb): > 250 µm maximal 1 Gew.-%, > 75 µm maximal 30 Gew.-%, > 32 µm mindestens 50 Gew.-%. pH 5,0 bis 7,0; Sulfatasche maximal 0,05 Gew.%. Vivapur^{®} 12 weist üblicherweise z.B. folgende allgemeine Spezifikation auf: Trocknungsverlust maximal 6 Gew.-%; Schüttdichte etwa 0,35 g/ml; Stampfvolumen etwa 1,9 ml/g; mittlere Korngröße 160 pm; Korngrößenverteilung: d₁₀: < 30 µm, d₅₀: 40 bis 70 µm, d₉₀: > 80 µm; Siebanalyse (Rückstand auf dem Luftstrahlsieb): 400 µm maximal 1 Gew.-%, 160 pm maximal 50 Gew.-%, 50 µm mindestens 70 Gew.-%.

Lactose ist ebenfalls als pharmazeutischer Grundstoff im Handel als ein weißes, gesiebtes, kristallines, geruchloses, in Wasser leicht und in Ethanol praktisch unlösliches Pulver erhältlich, z.B. als Capsulac^{®} (der Firma Meggle), insbesondere als Capsulac^{®} 60 oder Capsulac^{®} 200. Die im Handel als Capsulac^{®} 60 erhältliche Lactose für pharmazeutische Zwecke weist üblicherweise folgende Spezifikation auf: sauer oder alkalisch reagierende Substanzen maximal 0,4 ml 0,1 n Natronlauge; spezifische Drehung 54,4° bis 55,9°; Wasser (DAB) 4,5 bis 5,5 Gew.-%; Trocknungsverlust maximal 0,5 Gew.-%; Sulfatasche maximal 0,1 Gew.-%; Glührückstand maximal 0,1 Gew.-%; Korngrößenverteilung (Rüttelsiebung, 25 g, 10 Minuten): < 100 µm maximal 10 Gew.-%, < 630 µm mindestens 97 Gew.-%. Die im Handel als Capsulac^{®} 200 (Type EP D 80) erhältliche Lactose für pharmazeutische Zwecke weist üblicherweise folgende Spezifikation auf: sauer oder alkalisch reagierende Substanzen maximal 0,19 ml 0,1 n Natronlauge; spezifische Drehung 55,4°; Gesamtwasser 5,39 Gew.-%; Trocknungsverlust 0,17 Gew.-%; Sulfatasche 0,04 Gew.-%; Glührückstand 0,04 Gew.-%; Korngrößenverteilung (Luftstrahlsiebung, 10 g, 2 Minuten): < 32 µm 45 bis 75 Gew.-%, < 100 µm mindestens 90 Gew.-%.

In zweckmäßigen Ausgestaltungen können die erfindungsgemäßen Präformulierungen durch weitere Parameter charakterisiert werden, wie z.B. die Partikelgrößenverteilung, die mittlere bzw. durchschnittliche Partikelgröße, die Porosität der Partikel, das mittlere Schüttgewicht (Schüttdichte) und/oder mittlere Schüttvolumen.

Zweckmäßige erfindungsgemäße pharmazeutische Präformulierungen weisen z.B. ein mittleres Schüttvolumen im Bereich von 1,8 bis 3,0 ml/g auf. Das mittlere Schüttgewicht (Schüttdichte) der erfindungsgemäßen pharmazeutischen Präformulierung liegt z.B. im Bereich von 0,3 bis 0,6 g/ml. In einer Alternative zeichnet sich die erfindungsgemäße pharmazeutische Präformulierung dadurch aus, dass die Präformulierung eine durch Siebanalyse als prozentuale Durchgangssumme in Abhängigkeit von der Siebmaschenweite charakterisierte Partikelgrößenverteilung von 100 Gew.-% der Partikel bei einer Maschenweite von 500 µm, von wenigstens 98 Gew.-% der Partikel bei einer Maschenweite von 250 µm, von etwa 65 bis 99,5 Gew.-% der Partikel bei einer Maschenweite von 160 µm, von etwa 35 bis 87 Gew.-% der Partikel bei einer Maschenweite von 125 µm, und einem Feinanteil von unter 23 Gew.-% bei einer Maschenweite von 63 µm, jeweils bezogen auf die Gesamtsumme der Siebfraktionen als 100 Gew.-%, aufweist. Alternativ zeichnet sich die erfindungsgemäße pharmazeutische Präformulierung dadurch aus, dass die Präformulierung eine durch Siebanalyse in Abhängigkeit von der Siebmaschenweite charakterisierte Partikelgrößenverteilung von etwa 0,15 bis maximal 2 Gew.-% der Partikel größer als eine Maschenweite von 250 µm, von etwa 3 bis 31 Gew.-% der Partikel größer als eine Maschenweite von 160 µm, von etwa 8 bis 36 Gew.-% der Partikel größer als eine Maschenweite von 125 µm, und einem Feinanteil der Partikel von etwa 3 bis maximal 23 Gew.-% bei einer Maschenweite von 63 µm, jeweils bezogen auf die Gesamtsumme der Siebfraktionen als 100 Gew.-%, aufweist. Die mittlere (durchschnittliche) Partikelgröße der erfindungsgemäßen pharmazeutischen Präformulierung liegt zweckmäßigerweise im Bereich von 50 bis 250 µm, vorzugsweise im Bereich von 75 bis 150 µm.

Die vorliegende Erfindung betrifft weiterhin auch ein Verfahren zur Herstellung der vorstehend beschriebenen erfindungsgemäßen Trockenextrakte von natürlichen Gemischen konjugierter equiner Oestrogene, insbesondere von Gemischen aus dem Harn trächtiger Stuten gewonnener, konjugierter Oestrogene, wobei durch diese Trockenextrakte pharmazeutische Präformulierungen von natürlichen Gemischen konjugierter Oestrogene bereitgestellt werden, die sich für die Herstellung von festen galenischen Formen, z.B. zur Herstellung von Tabletten und insbesondere gewünschtenfalls auch für Direkttablettierung, eignen. Das Verfahren der Erfindung zur Herstellung der erfindungsgemäßen pharmazeutischen Präformulierung in Form eines festen, freifließenden Trockenextraktes der oben definierten Art für die Tablettierung zeichnet sich dadurch aus, dass man auf einen, in einem Wirbelschichtgerät fluidisierten pulver- und/oder granulatförmigen pharmazeutischen Trägerstoff, der aus der Gruppe mikrokristalliner Cellulosen oder einem Gemisch von mikrokristalliner Cellulose mit Lactose ausgewählt ist, eine wässrige Lösung, die ein Gemisch natürlicher konjugierter equiner Oestrogene als Wirkstoff enthält, in einer Menge aufsprüht, die dem in der pharmazeutischen Präformulierung erwünschten definierten, (bezogen auf die Haupthormon-Komponenten) standardisierten Wirkstoffgehalt entspricht, und man die erhaltenen wirkstoffhaltigen Partikel trocknet.

Die im Verfahren einsetzbaren mikrokristallinen Cellulose-Typen und Lactose-Typen sind weiter oben bereits im Zusammenhang mit den erfindungsgemäßen pharmazeutischen Präformulierungen beschrieben.

Für das erfindungsgemäße Verfahren können als CE-enthaltende, aus PMU beliebiger Herkunft gewonnene wässrige Lösungsextrakte in einem weiten Bereich varierender CE-Konzentration eingesetzt werden, die durch die weiter oben zum Stand der Technik beschriebenen Aufarbeitungsverfahren für PMU erhalten werden können, insbesondere durch das in der WO 98/08526 beschriebene oder ähnliche Verfahren unter Verwendung semipolarer, vorzugsweise nicht-ionischer semipolarer Adsorberharze. Je nach Konzentration der CE und der ggf. in diesen Extrakten verbliebenen Begleitstoffe können diese wässrigen Extrakte durch weiteres Entfernen von Lösungsmittel aufkonzentriert oder durch Zugabe von weiterem Wasser bzw. von mit Wasser mischbaren organischen Lösungsmitteln wie z.B. niederen aliphatischen Alkoholen auf gewünschte Wirkstoffgehalte für den Einsatz im Verfahren der vorliegenden Erfindung eingestellt werden.

In einer Variante des erfindungsgemäßen Verfahrens kann die eingesetzte Wirkstoff enthaltende wässrige Lösung somit neben dem Wasser noch andere, mit Wasser mischbare organische Lösungsmittel, insbesondere noch einen oder mehrere niedere aliphatische Alkohole, als zusätzliches Lösungsmittel enthalten. Geeignete niedere aliphatische Alkohole sind insbesondere solche mit ein bis vier Kohlenstoffatomen, beispielsweise Methanol, Ethanol, Isopropanol oder n-Butanol. Bevorzugt sind Methanol, Ethanol oder Isopropanol. Die organischen Lösungsmittel, insbesondere die Alkohole, können auch im Gemisch miteinander als zusätzliches Lösungsmittel der wässrigen Lösung zugesetzt sein. Die Menge des mit Wasser mischbaren organischen Lösungsmittelanteils, insbesondere des Alkoholanteils, kann in der wässrigen Lösung in den Bereichen liegen, wie diese in der WO 98/08526 als geeignet beschrieben sind. Andere ggf. geeignete mit Wasser mischbare Lösungsmittel wie Ketone oder wasserlösliche Ether sind ebenfalls in der WO 98/08526 beschrieben.

Bevorzugt werden im erfindungsgemäßen Verfahren allerdings Wirkstoff enthaltende wässrige Lösungen, d.h. CE-Extraktlösungen oder -konzentrate, eingesetzt, die eine weitgehend von organischem Lösemittel befreite, zur galenischen Weiterverarbeitung geeignete wässrige Lösung, d.h. ein weitgehend rein wässrige Lösung, der CE oder ein weitgehend von organischem Lösemittel befreites Konzentrat der CE sind. Sehr bevorzugt sind hierbei rein wässrige Lösungen oder Konzentrate des natürlichen Gemisches konjugierter Oestrogene.

Zweckmäßige Ausführungsvarianten des vorliegenden erfindungsgemäßen Verfahrens zeichnen sich dadurch aus, dass die eingesetzte wässrige Lösung einen Wirkstoffgehalt berechnet als Trockensubstanz des Gemisches natürlicher equiner konjugierter Oestrogene (Gesamthormongehalt einschließlich der freien Oestrogene und sonstiger Feststoffe) im Bereich von etwa 3,5 bis 20 Ges.-% bezogen auf die wässrige Lösung als 100_Gew.-% aufweist. Bevorzugt liegt der als Trockensubstanz des natürlichen Gemisches konjugierter equiner Oestrogene berechnete Wirkstoffgehalt in der wässrigen Lösung im Bereich von etwa 3,5 bis 14,5 Gew.-% bezogen auf die wässrige Lösung als 100 Gew.-%. Wird der Wirkstoffgehalt der erfindungsgemäß im Verfahren eingesetzten wässrigen Lösung als Gesamthormongehalt (einschließlich der freien Oestrogene) berechnet, so weist die eingesetzte wässrige Lösung einen Wirkstoffgehalt im Bereich von 10 bis 100 mg pro 1 g der wässrigen Lösung, vorzugsweise im Bereich von 10 bis 40 mg pro 1 g der wässrigen Lösung, auf.

Wird im erfindungsgemäßen Verfahren als wässrige Lösung ein Konzentrat eingesetzt, so weist dieses zweckmäßigerweise einen Wirkstoffgehalt berechnet als Trockensubstanz des Gemisches natürlicher konjugierter equiner Oestrogene (Gesamthormongehalt einschließlich der freien Oestrogene und sonstiger Feststoffe) im Bereich von größer 20 Gew.-% bezogen auf das Konzentrat als 100 Gew.-% auf. Wird der Wirkstoffgehalt des erfindungsgemäß im Verfahren eingesetzten wässrigen Konzentrates als Gesamthormongehalt (einschließlich der freien Oestrogene) des Gemisches natürlicher equiner konjugierter Oestrogene (CE) berechnet, so weist das eingesetzte Konzentrat zweckmäßigerweise einen Wirkstoffgehalt von größer 40 mg pro 1 g des Konzentrates (100 Gew.-%) auf.

Zweckmäßigerweise werden im erfindungsgemäßen Verfahren wässrige Lösungen eingesetzt, in denen der Gesamthormongehalt (einschließlich der freien Oestrogene) bezogen auf die in der wässrigen Lösung enthaltene Trockensubstanz als 100 Gew.-% im Bereich von 18 bis 31 Gew.-% liegt.

Das erfindungsgemäße Verfahren zur Herstellung der erfindungsgemäßen Trockenextrakte bzw. Präformulierungen von natürlichen Gemischen konjugierter Oestrogene, insbesondere von Gemischen aus dem Harn trächtiger Stuten gewonnener, konjugierter Oestrogene kann in an sich beliebigen, üblichen Wirbelschichttrocknungsgeräten, insbesondere solchen für den Einsatz im pharmazeutischen Betrieb, durchgeführt werden. Geeignete Wirbelschichtgeräte sind z.B. das Wirbelschichtgerät "Strea I". Im erfindungsgemäßen Verfahren wird der pulver- oder granulatförmigen pharmazeutischen Trägerstoff, z.B. die mikrokristalline Cellulose oder einem Gemisch von mikrokristalliner Cellulose mit Lactose, im Wirbelschichtgerät in einer vorberechneten Produktionsmenge vorgelegt und mittels eines Luftstromes fluidisiert. Anschließend wird eine ein natürliches Gemisch konjugierter Oestrogene als Wirkstoff enthaltende wässrige Lösung in einer Menge, die dem in der Präformulierung erwünschten Wirkstoffgehalt entspricht, auf den Trägerstoff aufsprüht und die erhaltenen wirkstoffhaltigen Partikel trocknet.

Das Verfahren kann hierbei sowohl kontinuierlich als auch diskontinuierlich im Chargenbetrieb durchgeführt sowie neben der Art und Menge des eingesetzten Trägerstoffes bzw. neben der Art, der Menge und dem Wirkstoffgehalt der eingesetzten wässrigen Lösung, weiterhin über dem Fachmann in der Wirbelschichttechnik geläufige Verfahrensparameter wie z.B. Zu- und Ablufttemperatur, Menge des zu- und abgeführten Luftstromes, die Aufsprühgeschwindigkeit der wässrigen Lösung sowie bei kontinuierlicher Verfahrensweise auch durch die Geschwindigkeit des Feststoffeintrages und Produktaustrages bzw. der Verweildauer des Produktes im Wirbelschichtgerät gesteuert werden.

In einer zweckmäßigen Variante des erfindungsgemäßen Verfahrens liegt z.B. die anhand der Ablufttemperatur regulierte Temperatur des im Wirbelschichtgerät fluidisierten Präformulierungs-Produktes im Bereich von 25 bis 60 °C, vorzugsweise im Bereich von 45 bis 55 °C. In einem Ausführungsbeispiel des erfindungsgemäßen Verfahrens liegt die anhand der Ablufttemperatur regulierte Temperatur des im Wirbelschichtgerät fluidisierten Präformulierungs-Produktes bei etwa 45 bis 55 °C.

In einer zweckmäßigen Variante des erfindungsgemäßen Verfahrens liegt z.B. die über die relative Abluftfeuchte regulierte Prozeßfeuchtigkeit im Wirbelschichtgerät im Bereich von 50 bis 80 % r.h. (r.h. = relative humidity bzw. relative Feuchte).

In einer zweckmäßigen Variante des erfindungsgemäßen Verfahrens wird z.B. die eingesetzte Wirkstoff enthaltende wässrige Lösung mit einer Sprühgeschwindigkeit von 20 bis 50 g/min, auf den im Wirbelschichtgerät fluidisierten pulver- und/oder granulatförmigen pharmazeutischen Trägerstoff aufgesprüht.

Im erfindungsgemäßen Verfahren zur Herstellung der erfindungsgemäßen Trockenextrakte bzw. Präformulierungen von natürlichen Gemischen konjugierter Oestrogene, insbesondere von Gemischen aus dem Harn trächtiger Stuten gewonnener, konjugierter Oestrogene werden in zweckmäßigen Ausführungsvarianten pulver- und/oder granulatförmige Trägerstoffe eingesetzt, die durch bestimmte Partikeleigenschaften gekennzeichnet sind und somit zur gezielten Steuerung der Partikeleigenschaften des Trockenextrakt- bzw. Präformulierungsproduktes verwendet werden können. Als geeignete Parameter für die Partikeleigenschaften der eingesetzten pulver- bzw. granulatförmigen Trägerstoffe kommen ebenso wie für die Charakterisierung der auf dieser Basis hergestellten Trockenextrakt- bzw. Präformulierungsprodukte z.B. die Partikelgrößenverteilung, die mittlere bzw. durchschnittliche Partikelgröße, die Porosität der Partikel oder das mittlere Schüttgewicht sowie vom Fachmann im konkreten Fall als zweckmäßig erachtete weitere Parameter herangezogen werden. Nachfolgend werden zur Orientierung einige zweckmäßige Bereiche dieser Partikelparameter angegeben.

In einer vorteilhaften Variante des erfindungsgemäßen Verfahren wird ein pulver- und/oder granulatförmiger pharmazeutischer Trägerstoff, insbesondere eine mikrokristalline Cellulose, eingesetzt, der eine durch Siebanalyse als prozentuale Durchgangssumme in Abhängigkeit von der Siebmaschenweite charakterisierte Partikelgrößenverteilung von 100 Gew.-% der Partikel bei einer Maschenweite von 500 µm, von wenigstens 99 Gew.-% der Partikel bei einer Maschenweite von 250 µm, von etwa 85 bis 95 Gew.-% der Partikel bei einer Maschenweite von 160 µm, von etwa 70 bis 80 Gew.-% der Partikel bei einer Maschenweite von 125 pm, und einem Feinanteil von bis zu etwa 50 Gew.-% bei einer Maschenweite von 63 µm, jeweils bezogen auf die Gesamtsumme der Siebfraktionen als 100 Gew.-%, aufweist. Besonders zweckmäßige im erfindungsgemäßen Verfahren eingesetzte pulver- und/oder granulatförmige pharmazeutische Trägerstoffe, insbesondere die mikrokristalline Cellulose, weisen hierbei eine mittlere (durchschnittliche) Partikelgröße im Bereich von 50 bis 130 µm auf. Der im erfindungsgemäßen Verfahren eingesetzte pulver- und/oder granulatförmige pharmazeutische Trägerstoff, insbesondere die mikrokristalline Cellulose, weist z.B. ein Schüttgewicht (Schüttdichte) im Bereich von etwa 25 bis 35 g/ml auf. Weiterhin sind die im erfindungsgemäßen Verfahren eingesetzten pulver- und/oder granulatförmigen pharmazeutischen Trägerstoffe, insbesondere die mikrokristalline Cellulose, dadurch gekennzeichnet, dass der Wassergehalt (Trocknungsverlust) maximal etwa 6 Gew.-% beträgt.

Nach dem erfindungsgemäßen Verfahren wird in vorteilhafter Weise ein für die Herstellung von Pharmazeutika, die das natürliche Gemisch konjugierter Oestrogene aus dem PMU als Wirkkomponente enthalten, dienendes Ausgangsmaterial bereitgestellt, das sich als Trockenextrakt bzw. Präformulierung von hervorragender Qualität vorteilhaft für die Weiterverarbeitung durch Direkttablettierung eignet.

Das erfindungsgemäße Verfahren und die erfindungsgemäße Präformulierung weisen eine Reihe von Vorteilen insbesondere auch gegenüber anderen Verfahrensweisen auf. Es können CE-enthaltende wässrige Extrakte mit geringer Hormonkonzentration verarbeitet werden. Im Gegensatz zu Beobachtungen bei konventioneller Sprühtrockung solcher CE-enthaltender Extrakte werden beim erfindungsgemäßen Verfahren in der Wirbelschicht unerwünschte Anhaftungen z.B. an den Düsen nicht beobachtet. Die thermische Belastung der wertvollen Hormonbestandteile der eingesetzten wässrigen Extrakte ist beim erfindungsgemä-βen Verfahren in der Wirbelschicht sehr gering. Klebrige Eigenschaften, z.B. Verklumpung, des CE-enthaltenden wässrigen Extraktes kommen weniger zum Tragen als bei anderen Trocknungsverfahren wie z.B. bei Single-Pot Technologie. Gegenüber Arbeitsweisen in Vakuumtrocknern etc. ist das erfindungsgemä-βen Verfahren ein kontinuierlich durchführbares Verfahren, dass zudem - sowohl bei kontinuierlicher als auch diskontinuierlicher Verfahrensweise - den Auftrag großer Flüssigkeitsmengen, auch ohne Überfeuchtung, erlaubt. Im erfindungsgemä-βen Verfahren kann ein weites Spektrum an Extrakten sowohl hinsichtlich der Hormonkonzentration als auch der Konzentration an Begleitstoffen verarbeitet werden. Dadurch kann das Verfahren sehr gut die aufgrund der natürlichen Schwankungen des PMU-Ausgangsmaterials in der großtechnischen Praxis zu bewältigenden Probleme lösen. Es zeigte sich, dass durch Aufsprühen eines Hormonkonzentrates mit Hilfe der Wirbelschichttechnik auf erfindungsgemäß verwendete Trägerstoffe, wie mikrokristalline Cellulose oder ggf. Gemische von mikrokristalliner Cellulose mit Lactose, die konjugierten Hormone homogen auf die Trägerstoffe aufgetragen werden können. Die nach dem erfindungsgemäßen Verfahren hergestellten Präformulierungen in Form fester, freifließender Trockenextrakte sind sehr stabile pulver- bzw. partikelförmige hormonhaltige Produkte, die überraschend gut in Matrixtabletten homogen verteilt und verpreßt werden können. Somit sich lassen sich aus den erfindungsgemäßen pharmazeutischen Präformulierungen in einfacher Weise Matrixtabletten mit einem gewünschten Freisetzungsprofil fertigen.

Die nachfolgenden Beispiele sollen die Erfindung näher erläutern, ohne jedoch ihren Umfang zu beschränken.

### Beispiele

### Beispiel 1:

### Trocknung bzw. Herstellung einer Präformulierung mit hormonhaltigem Wirkstoff in einem Wirbelschichtgerät, und Hormonbilanz

Es wurden eine Reihe von Versuchen mit dem Ziel der Entwicklung eines hormonhaltigen Wirkstoffs durch Trocknung der Hormone aus Urinkonzentrat von trächtigen Stuten durchgeführt. Die konjugierten Hormone mußten hierbei in eine Form gebracht werden, welche die chemische Stabilität der Hormone gewährleistet und die Verarbeitung der Hormone in einer Tablette ermöglicht. Es wurde ein Urinkonzentrat (konzentrierte wässrige Lösung von Urin trächtiger Stuten = PMU) aus einer Sammelkampagne in Asien eingesetzt, das durch seine Menge an Trockensubstanz und Hormonkonzentration charakterisiert war. Das Urinkonzentrat wurde vor der Verwendung gemäß dem Verfahren der WO 98/08526 aufgearbeitet, um unerwünschte Begleitstoffe wie Harnstoff, HPMF und Kresole abzutrennen.

In den Versuchen zeigte sich, dass durch Aufsprühen des Hormonkonzentrates durch die Wirbelschichttechnik auf Trägerstoffe wie mikrokristalline Cellulose oder Gemische aus mikrokristalliner Cellulose mit Lactose die konjugierten Hormone auf die Hilfsstoffe homogen aufgetragen werden konnten.

Ein aus einer Sammelkampagne bereitgestelltes Urinkonzentrat wurde auf mikrokristalline Cellulose oder auf ein Gemisch von mikrokristalliner Cellulose und Lactose aufgesprüht und dadurch die Hormone auf den Träger oder das Trägerstoffgemisch aufgetragen. Dieser Prozeß wurde in einem Wirbelschichtgranulator durchgeführt. Die Partikelgröße und Porosität des Wirkstoffgranulats wurde durch die Zuluft- und Ablufttemperatur und die Sprühgeschwindigkeit reguliert. Als Parameter für den Prozeß dienten die Produkttemperatur (reguliert an Hand der Ablufttemperatur), die im Bereich von 25 bis 55 °C eingestellt war, sowie die Prozeßfeuchtigkeit (reguliert über relative Abluftfeuchte), die im Bereich von 50 bis 80 % relative Luftfeuchte eingestellt war. Die Sprühgeschwindigkeit wurde entsprechend gewählt, um die vorgenannten Bereiche einzuhalten.

Zur Herstellung von Trockenextrakten von natürlichen Gemischen konjugierter Oestrogene wurde in diesen Versuchen ein Wirbelschichtgerät (Strea 1) eingesetzt, mit dem ca. 1 kg Trockenextrakt pro Ansatz hergestellt werden kann. Der wässrige, ein natürliches Gemisch konjugierter Oestrogene enthaltender Lösungsextrakt wurde nach dem Top-Spray-Verfahren in das Wirbelschichtgerät eingebracht. Die weitere technische Ausrüstung umfaßte:
- Sartorius Waage / 6,2 kg / Typ LC6200S-OD2,
- Schlauchpumpe Masterflex 07523-27 mit Pumpenkopf 7518-10,
- Feuchtemeßgerät vom Typ HR 73 von Mettler Toledo.

Die Versuche im Wirbelschichtgerät wurden mit wässrigen, ein natürliches Gemisch konjugierter Oestrogene enthaltenden Lösungsextrakten durchgeführt, die aus einer gemäß dem in der WO 98/08526 beschriebenen Verfahren aufgearbeiteten Sammelkampagne in Asien entstammten, wobei die hormonhaltigen wässrigen Extrakte die folgenden Hormongehalte aufwiesen: Versuch 1: TS = 9,2 Gew.-%
Versuch 2: TS = 15,9 Gew.-%
Versuch 3: TS = 19,3 Gew.-%
Versuch 4: TS = 9,2 Gew.-%
In weiteren Versuchen wurden CE-enthaltende wässrige Lösungsextrakte mit TS = 11,8 Gew.-% (Versuch 5) bzw. TS = 9,9 Gew.-% (Versuch 6) eingesetzt. Die wässrigen Lösungsextrakte wiesen alle einen kristallinen oder öligen Niederschlag auf, der eine homogene Verarbeitung aber nicht wesentlich beeinträchtigte. Die wässrigen Lösungsextrakte besaßen nur einen relativ niedrigen Hormongehalt, weshalb die Trockenextrakte auf einen theoretischen Soll-Gehalt von 45 mg konjugierte Oestrogene pro g Trockenextrakt eingestellt wurden.

Als Trägerstoffe für das natürliche Gemisch konjugierter Oestrogene wurden eingesetzt:
- Avicel PH 102,
- Capsulac 60.

### VERSUCHSDURCHFÜHRUNG

Herstellung eines Trockenextraktes mit einem Gehalt von 45 mg konjugierter Oestrogene pro g Trockenextrakt bei Vorlagen von 570 bis 680 g des Trägerstoffes.

### Versuch 1:

| | |
|---|---|
| eingesetzter Extrakt: | 4023,1 g; TS = 9,2 Gew.-%; |
| Dichte: | 1,0365 g/l; CE = 12,14 g/l |
| Vorlage: | 677,0 g Avicel PH 102 |
| Sprührate: | 40-50 g/min (ungefährer Mittelwert) |
| relative Abluftfeuchte: | 70-80 % |
| Ablufttemperatur: | 32-34 °C |

### Versuch 2 :

| | |
|---|---|
| eingesetzter Extrakt: | 2400,0 g; TS = 15,9 Gew.-%; |
| Dichte: | 1,0662 g/l; CE = 20,86 g/l |
| Vorlage: | 661,9 g Avicel PH 102 |
| Sprührate: | 40-50 g/min (ungefährer Mittelwert) |
| relative Abluftfeuchte: | 70-80 % |
| Ablufttemperatur: | 32-34 °C |

### Versuch 3 :

| | |
|---|---|
| eingesetzter Extrakt: | 1904,6 g; TS = 19,3 Gew.-%; |
| Dichte: | 1,0662 g/l; CE = 20,86 g/l |
| Vorlage: | 574,8 g Avicel PH 102 |
| Sprührate: | 40-50 g/min (ungefährer Mittelwert) |
| relative Abluftfeuchte: | 70-80 % |
| Ablufttemperatur: | 32-34 °C |

Alle 3 Versuche verliefen unproblematisch. Die Sprühzeiten lagen bei Versuch 1 bei 83 Minuten, bei Versuch 2 bei 46 Minuten und bei Versuch 3 bei 35 Minuten.

### Versuch 4 :

| | |
|---|---|
| eingesetzter Extrakt: | 4023,1 g; TS = 9,2 Gew.-%; |
| Dichte: | 1,0365 g/l; CE = 12,14 g/l |
| Vorlage: | 677,0 g Avicel PH 102 |
| Sprührate: | 40-50 g/min (ungefährer Mittelwert) |
| relative Abluftfeuchte: | 50-60 % |
| Ablufttemperatur: | 35-40 °C |

Dieser Versuch ist eine Wiederholung von Versuch 1, durch den Überprüft werden sollte, ob durch Verringerung der Sprührate ein feineres Trockenextrakt hergestellt werden kann. Der Trockenextrakt erwies sich in Siebanalysen gegenüber dem in Versuch 1 erhaltenen Trockenextrakt als feiner (siehe Zusammenfassung der Ergebnisse der Versuche).

Weitere Versuche wurden in analoger Verfahrensweise zu den Versuchen 1 bis 3 mit Avicel PH 102 (Versuch 5) oder mit Gemischen von Avicel PH 102 und Capsulac 60 (Gewichtsverhältnis 7:3; Versuch 6) durchgeführt.

### VERSUCHSERGEBNISSE

Detailergebnisse zur Hormonbilanz in den Versuchen 1 bis 4 sind in den Tabellen I bis IV zusammengefaßt.

Prinzipiell wurde gefunden, dass bei einer Vorlage von 570 g bis 680 g Avicel PH 102 als Trägerstoff ein kontinuierlicher und schneller Auftrag des Extraktes möglich ist (Versuche 1 bis 3). Für die eingesetzten von 1900 bis 4023 g variierten Extraktmengen lagen die Sprühzeiten für diese Versuche zwischen 35 und 83 Minuten. Hieraus ergaben sich Auftragsmengen von 0,55 g bis 0,64 g Feststoff aus dem Extrakt pro g Avicel (Mittelwert: 0,59 g).

Um in einem weiteren Versuch (Versuch 5) den vorgegebenen Soll-Gehalt von 45 mg konjugierte Oestrogene pro g Trockenextrakt einzuhalten bzw. Grenzen für maximal auftragbare Wirkstoffmengen zu ermitteln, wurde in diesem Versuch die Vorlage an Avicel PH 102 auf 342,5 g reduziert - gegenüber den vorherigen Versuchen 1 bis 4 also eine Reduzierung um fast 50%. Aufgetragen werden sollte 4640 g Extrakt. Hierbei traten bis ca. 1600 g aufgesprühtem Extrakt keine Probleme auf, da bis zu dieser Menge wie in den vorangegangenen Versuchen 1 bis 4 wieder eine Auftragsmenge von 0,56 g Feststoff aus dem Extrakt pro g Avicel vorlag. Bei ca. 2000 g aufgesprühtem Extrakt ergab sich eine Auftragsmenge von 0,68 g und bei ca. 2500 g von 0,86 g Feststoff aus dem Extrakt pro g Avicel. Bis zu dieser Auftragsmenge ließ sich der Extrakt weitgehend unproblematisch aufsprühen. Danach wurde die Sprührate stark reduziert, da ab dieser Menge der Feststoff aus dem Extrakt die Menge des Trägerstoffes übersteigt und das Produkt ab diesem Punkt Klebeneigung zeigte. Der Prozeß wurde dann nur noch bei einer relativen Feuchte von < 25 % gefahren, da sich die Abluftfilter zusetzten; die Luftmenge war nicht mehr ausreichend um das Wirbelbett aufrecht zu erhalten. Die reine Sprühzeit betrug mehr als 5 Stunden.

Zusammenfassend kann daher gesagt werden, dass bis zu einem Auftrag von 0,6 g Feststoff aus dem Extrakt pro g Avicel der gesamte Extrakt verarbeitet sein sollte. Bei etwa 0,86 g Auftrag an Feststoff aus dem Extrakt liegt die Mengen-Obergrenze, die Avicel PH 102 an Extrakt ohne Beeinträchtigung aufnehmen kann. Danach ist eine Reduktion der Sprühauftrages und eine entsprechende Anpassung der restlichen Parameter erforderlich.

Versuch 4 stellt eine Wiederholung von Versuch 1 dar. Hier wurde durch Veränderung der Parameter (niedrigere Sprührate und dadurch höhere Ablufttemperatur und eine geringere Abluftfeuchte) eine feinere Verreibung hergestellt.

Beim zusätzlichen Versuch 6 erfolgte wie in Versuch 4 eine Reduktion der Vorlage, um auf 45 mg konjugierte Estrogene pro g Verreibung einstellen zu können (Reduktion > 60% gegenüber Versuch 1 und Versuch 2). Zusätzlich wurde hier Lactose eingesetzt (Verhältnis Avicel zu Lactose = 7 zu 3). In diesem Versuch war ab einer Auftragsmenge von 0,6 g Feststoff aus dem Extrakt pro g Gemisch Avicel/Lactose eine Absenkung der Sprührate von 20 g/min auf < 9 g/min erforderlich (bei V-140 lag die Grenze bei 0,86 g Feststoff). Die aufgesprühte Extraktmenge betrug zu diesem Zeitpunkt ca. 40 Gew.-% (< 1600 g). Ab ca. 1800 g trat auch hier, wie bereits in Versuch 4 beobachtet, Klebeneigung auf. Der Versuch wurde nach Auftrag von 70 % Extraktmenge abgebrochen, da eine weitere Absenkung der Sprührate (< 9 g/min) auf Grund der Geräte nicht möglich war.

**Tabelle I**

| | | | | | | | | |
|---|---|---|---|---|---|---|---|---|
| Hormonbilanz für den Versuch 1 | | | | | | | | |

| **Oestrogene** | **Gesamtestrogene** | | | | **Freie Estrogene :** | | | |
|---|---|---|---|---|---|---|---|---|
| | Lösungsextrakt | | Trockenextrakt | | Lösungsextrakt | | Trockenextrakt | |
| | [mg/gj | [%]¹⁾ | [mg/g] | [%]¹⁾ | [mg/g] | [%]²⁾ | [m^{g}/g] | [%]²⁾ |
| 17-α-Estradiol | 0,455 | 4,34 | 1,856 | 4,31 | 0,047 | | 0,196 | |
| 17-β-Estradiol | 0,646 | 6,17 | 2,631 | 6,12 | 0,082 | | 0.344 | |
| 17-α-DH-Equilin | 1,270 | 12,12 | 5,160 | 11,99 | 0,098 | 0,94 | 0,405 | 0,94 |
| 17-β-DH-Equilin | 0,322 | 3,07 | 1,323 | 3,08 | 0,019 | | 0,077 | |
| 17-α-DH-Equilenin | 0,057 | 0,54 | 0,229 | 0,53 | 0,007 | | 0,021 | |
| 17-β-DH-Equilenin | 0,031 | 0,30 | 0,215 | 0,50 | 0,000 | | 0.000 | |
| Estron | 6,193 | 59,12 | 25,371 | 58,971 | 0,2471 | 2,36 | 1,015 | 2,36 |
| Equilin | 2,236 | 21,34 | 9,312 | 21,64 | 0,057 | 0,54 | 0,229 | 0,53 |
| δ-8,9-Dehydroestron | 0,293 | 2,80 | 1,223 | 2,84 | 0,022 | | 0,076 | |
| Equilenin | 0,124 | 1_{;}18 | 0.515 | 1.20 | 0,000 | | 0,000 | |
| Gesamthormongehaft | 11,627 | | 47,835 | | 0,579 | | 2,363 | |
| Summe Haupthormone³⁾ | 10,476 | | 43,022 | | 0,468 | | 1,922 | |

| | | | | | | | | |
|---|---|---|---|---|---|---|---|---|
| 1) bezogen auf 17-α-Estradiol, 17-α-DH-Equilin, 17-β-DH-Equilin, Estron und Equilin 2) bezogen auf Summe 17-α-Estadiol, 17-α-DH-Equilin, 17-β-DH-Equilin, Estron und Equilin aus Gesamtestrogenen 3) Summe der Hormone 17-α-Estradiol, 17-α-DH-Equilin, 1T-β-DH-Equilin, Estron, Equilin | | | | | | | | |

**Tabelle II**

| Hormonbilanz für den Versuch 2 | | | | | | | | |
|---|---|---|---|---|---|---|---|---|
| **Oestrogene** | **Gesamtestrogene** | | | | **Freie Estrogene** | | | |
| | Lösungsextrakt | | Trockenextrakt | | Lösungsextrakt | | Trockenextrakt | |
| | [mg/g] | [%]¹⁾ | [mg/g] | [%]¹⁾ | [mg/g] | [%]²⁾ | [mg/g] | [%]²⁾ |
| 17-α-Estradiol | 0,826 | 4,46 | 2,085 | 4,49 | 0,087 | | 0,223 | |
| 17-β-Estradid | 1,220 | 6,58 | 3,074 | 6,62 | 0,163 | | 0,417 | |
| 17-α-DH-Equilin | 2,302 | 12,42 | 5,824 | 12,54 | 0,177 | 0.96 | 0,437 | 0,94 |
| 17-β-DH-Equilin | 0,634 | 3,42 | 1,506 | 3,24 | 0,036 | | 0,087 | |
| 17-α-DH-Equitenin | 0,124 | 0,57 | 0,298 | 0.64 | 0,012 | | 0,030 | |
| 17-β-DH-Equilenin | 0,103 | 0,56 | 0,242 | 0,52 | 0,000 | | 0,000 | |
| Estron | 10,835 | 58,47 | 27,056 | 58,28 | 0,423 | 2,28 | 1,056 | 2,27 |
| Equilin | 3,934 | 21,23 | 9,957 | 21,45 | 0,092 | 0,50 | 0,223 | 0,50 |
| δ-8,9-Dehydroestron | 0,529 | 2,85 | 1,348 | 2,90 | 0,016 | | 0,079 | |
| Equilenin | 0,220 | 1,19 | 0,543 | 1,17 | 0,000 | | 0.000 | |
| Gesamthomlongehalt | 20,727 | | 51,933 | | 1,006 | | 2,562 | |
| Summe Haupthormone³⁾ | 18,531 | | 46,428 | | 0,815 | | 2,036 | |

| | | | | | | | | |
|---|---|---|---|---|---|---|---|---|
| 1) bezogen auf 17-α-Estradiol, 17-α-DH-Equilin, 17-β-DH-Equilin, Estron und Equilin 2) bezogen auf Summe 17-α-Estradiol, 17-α-DH-Equilin, 17-β-DH-Equilin, Estron und Equilin aus Gesamtestrogenen 3) Summe der Hormone 17-α-Estradiol, 17-α-DH-Equilin, 17-β-DH-Equilin, Estron, Equilin | | | | | | | | |

**Tabelle III**

| Hormonbilanz für den Versuch 3 | | | | | | | | |
|---|---|---|---|---|---|---|---|---|
| **Oestrogene** | **Gesamtestrogene** | | | | **Freie Estrogene** | | | |
| | Lösungsextrakt | | Trockenextrakt | | Lösungsextrakt | | Trockenextrakt | |
| | [mg/g] | [%]¹⁾ | [mg/g] | [%]¹⁾ | [mg/g] | [%]²⁾ | [mg/g] | [%]²⁾ |
| 17-α-Estradiol | 1,003 | 4,64 | 2,208 | 4,57 | 0,102 | | 0,227 | |
| 17-β-Estradiol | 1,402 | 6,49 | 3,072 | 6,36 | 0,166 | | 0,365 | |
| 17-α-DH-Equilin | 2,678 | 12,40 | 5,984 | 12,39 | 0,207 | 0,96 | 0,463 | 0,96 |
| 17-β-DH-Equilin | 0,633 | 2,93 | 1,432 | 2,96 | 0,038 | | 0,091 | |
| 17-α-DH-Equilenin | 0,118 | 0,55 | 0,232 | 0,48 | 0,021 | | 0,031 | |
| 17-β-DH-Equilenin | 0,045 | 0,21 | 0,057 | 0,12 | 0,000 | | 0,000 | |
| Estron | 12,713 | 58,87 | 28,105 | 58,18^{.} | 0,492 | 2,28 | 1,083 | 2,24 |
| Equilin | 4,569 | 21,16 | 10,582 | 21,90 | 0,107 | 0,50 | 0,241 | 0,50 |
| δ-8,9-Dehydroestron | 0,539 | 2,50 | 1,265 | 2,62 | 0,021 | | 0,113 | |
| Equilenin | 0,222 | 1,03 | 0,492 | 1,02 | 0,000 | | 0,000 | |
| Gesamthormongehalt | 23,922 | | 53,429 | | 1,154 | | 2,614 | |
| Summe Haupthormone³⁾ | 21,596 | | 48,311 | | 0,946 | | 2,105 | |

| | | | | | | | | |
|---|---|---|---|---|---|---|---|---|
| 1) bezogen auf 17-α-Estradiol, 17-α-DH-Equilin, 17-β-DH-Equilin, Estron und Equilin 2) bezogen auf Summe 17-α-Estradiol, 17-α-DH-Equilin, 17-β-DH-Equilin, Estron und Equilin aus Gesamtestrogenen 3) Summe der Hormone 17-α-Estradiol, 17-α-DH-Equilin, 17-β-DH-Equilin, Estron, Equilin | | | | | | | | |

**Tabelle IV**

| Hormonbilanz für den Versuch 4 | | | | | | | | |
|---|---|---|---|---|---|---|---|---|
| **Oestrogene** | **Gesamtestrogene** | | | | **Freie Estrogene** | | | |
| | Lösungsextrakt | | Trockenextrakt | | Lösungsextrakt | | Trockenextrakt | |
| | [mg/g] | [%]¹⁾ | [mg/g]¹⁾ | [%] | [mg/g] | [%]²⁾ | [mg/g] | [%]²⁾ |
| 17-α-Estradiol | 0,646 | 4,56 | 2,284 | 4,56 | 0,057 | | 0,201 | |
| 17-β-Estradiol | 1,093 | 7,71 | 3,798 | 7,58 | 0,151 | | 0,531 | |
| 17-α-DH-Equilin | 1,876 | 13,23 | 6,367 | 12,71 | 0,134 | 0,94 | 0,484 | 0,97 |
| 17-β-DH-Equilin | 0,523 | 3,69 | 1,768 | 3,53 | 0,018 | | 0,109 | |
| 17-α-DH-Equilenin | 0,070 | 0,49 | 0,274 | 0,55 | 0,008 | | 0,030 | |
| 17-β-DH-Equilenin | 0,000 | 0,00 | 0,103 | 0,21 | 0,000 | | 0,000 | |
| Estron | 8,022 | 56,57 | 28,947 | 57,77 | 0,282 | 1,99 | 1,038 | 2,07 |
| Equilin | 3,114 | 21,96 | 10,743 | 21,44 | 0,068 | 0,50 | 0,229 | 0,46 |
| δ-8,9-Dehydroestron | 0,381 | 2,69 | 1,339 | 2,67 | 0,022 | | 0,083 | |
| Equilenin | 0,119 | 0,84 | 0,543 | 1,08 | 0,000 | | 0,000 | |
| Gesamthormongehalt | 15,844 | | 56,166 | | 0,740 | | 2,705 | |
| Summe Haupthormone³⁾ | 14,181 | | 50,109 | | | | 2,057 | |

| | | | | | | | | |
|---|---|---|---|---|---|---|---|---|
| 1) bezogen auf 17-α-Estradiol, 17-α-DH-Equilin, 17-β-DH-Equilin, Estron und Equilin 2) bezogen auf Summe 17-α-Estradiol, 17-α-DH-Equilin, 17-β-DH-Equilin, Estron und Equilin aus Gesamtestrogenen 3) Summe der Hormonen 17-α-Estradiol, 17-α-DH-Equilin, 17-β-DH-Equilin, Estron, Equilin | | | | | | | | |

### BEWERTUNG DER VERSUCHSERGEBNISSE

Die Herstellung eines Trockenextraktes im Wirbelschichtgerät, auch mit Trägerstoffen unterschiedlicher Korngrößenverteilung, ist unproblematisch. Die Ausbeutebestimmung lag bei allen Versuchen zwischen 90 und 95%. Die Hormonverteilung, bezogen auf 17-α-DH-Equilin, Estron und Equilin, ist im Extrakt und in der Verreibung gleichbleibend. Der Trocknungsprozeß hat also keinen Einfluß auf die Stabiltät der Hormone. Der Restfeuchtegehalt lag zwischen 3 und 6 % r.h..

Wie die Versuche zeigen, ist es möglich große Hormon-haltige Extraktmengen von 2 bis 4 kg innerhalb kurzer Zeit zu verarbeiten, d.h. auf Trägerstoffe aufzutragen und entsprechend zu trocknen. Als besonders wichtig hervorzuheben sind die ermittelten Auftragsmengen (g Feststoff aus dem Extrakt pro g Trägerstoff, z.B. Avicel), die maximal ohne Verfahrensprobleme auf den gewählten Trägerstoff aufgebracht werden können. Hier wurde festgestellt, dass bei Vorlage von z.B. Avicel als Trägerstoff ein Auftrag bis etwa 0,6 g Wirkstoff-Trockensubstanz aus dem hormon-haltigen wässrigen Lösungsextrakt pro g Avicel völlig problemlos ist (Versuche 1, 2 und 3 des Beispiels 1).

### Beispiel 2:

### Herstellung von Trockenextrakten unter Verwendung von wässrigen Hormonextraktlösungen mit variierendem Hormongehalt

Es wurden weitere Sprühversuche durchgeführt, um ein wässriges Lösungsextrakt, enthaltend natürliche Gemische equiner konjugierter Oestrogene in verschiedenen Konzentrationen, auf einen aus der Gruppe mikrokristalliner Cellulosen ausgewählten pharmazeutischen Trägerstoff aufzutragen. Die Sprühversuche wurden analog zum Beispiel 1 in einem Wirbelschichtsprühgerät mit 6 verschiedenen wässrigen Lösungsextrakten A bis F durchgeführt. Die Eigenschaften der eingesetzten wässrigen Lösungsextrakte A bis F (Wirkstoff-Lösungen) sowie die jeweils verwendeten Verfahrensparameter sind aus Tabelle V ersichtlich. Weitere Eigenschaften der in Form eines festen, freifließenden Trockenextraktes erhaltenen pharmazeutischen Präformulierungen sind in Tabelle VI angegeben.

Die in Form eines festen, freifließenden Trockenextraktes erhaltenen pharmazeutischen Präformulierungen wurden noch einer Siebanalyse unterzogen. Die Siebanalyse wurde mit einer Retsch-Siebmaschine unter folgenden Bedingungen durchgeführt: 5 min. Siebzeit, Impulszeit 3 s, Schwingungshöhe 1,50 mm, Probenmenge jeweils ca. 20 g. Es wurden Siebe mit einer Maschenweite ab 1000 µm eingesetzt. Die Ergebnisse der Siebanalysen sind in der Tabelle VII wiedergegeben.

**Tabelle V**

| **Herstellung von Trockenextraktfraktionen, enthaltend natürliche Gemische equiner konjugierter Oestrogene, Versuche im Wirbelschichtsprühtrockner** | | | | | | | | | | | | |
|---|---|---|---|---|---|---|---|---|---|---|---|---|
| **Wirkstoff-Lösungen** | **A** | | | **B** | | | **C** | | **D** | | **E** | **F** |
| TS[%] | 3,5 | | | 7,4 | | | 12,7 | | 14,5 | | 6,6 | 13,6 |
| CE [mg/g]: | 10,726 | | | 17,655 | | | 33,451 | | 26,274 | | 16,229 | 38,224 |
| CE/TS [%] | 30,65 | | | 23,9 | | | 26,34 | | 18,12 | | 24,59 | 28,11 |
| **"TEF" Ch.-B.:** | **V-A -1** | **V-A-2** | **V-A-3** | **V-B-1** | **V-B-2** | **V-B-3** | **V-C-1** | **V-C-2** | **V-D-1** | **V-D-2** | **V-E** | **V-F** |
| Ansatz [g]: | 2300,00 | 2300,00 | 986,56 | 2295,08 | 2295,08 | 2295,08 | 2295,08 | 2166,23 | 2265,40 | 2265,40 | 2321,61 | 2335,91 |
| Konz. Extraktisg. [g] | 18227,0 | 18227,0 | 8278,0 | 11049,0 | 11,049,0 | 11049,0 | 5833,2 | 5504,4 | 7760,0 | 7760,0 | 12224,0 | 5500,0 |
| Produkttemp. [°C] | 45-50 | 60 | 45-50 | 45-50 | 45-50 | 45-50 | 45-50 | 45-50 | 55 | 45-50 | 45-50 | 46-50 |
| Zulufttemp. [°C] | 117-121 | 114-121 | 112-120 | 91-119 | 10,0-119 | 118-121 | 102-121 | 106-120 | 94-117 | 86-118 | 96-120 | 87-116 |
| Ablufttemp. [°C] | 43-45 | 50-57 | 42-43 | 42-47 | 39-47 | 39-48 | 38-46 | 43-45 | 50-53 | 44-46 | 43-46 | 45-50 |
| Abluftfeuchte [% r.F.] | 23-36 | 14-17 | 27-31 | 21-29 | 19-29 | 22-35 | 21-36 | 25-31 | 17-19 | 19-29 | 24-32 | 28-36 |
| Sprührate [g/min] | 34-40 | 20-30 | 21-34 | 30-39 | 30-46 | 37-43 | 36-45 | 30-42 | 40-52 | 27-37 | 32-36 | 30 |
| Luftmenge [m³/h] | 80-100 | 80-100 | 70-85 | 80-100 | 80-100 | 80-100 | 80-90 | 80-95 | 110-170 | 80-130 | 80-100 | 80-100 |
| Sprühdauer [h:m] | 07:58 | 12:17 | 04:25 | 02:05 | 04:50 | 04:43 | 02:40 | 02:30 | 01:05 | 04:18 | 06:03 | 03:25 |
| mit Prozessunterbrechung [J/N] | J | J | N | J | N | N | N | N | J | J | J | N |
| Restfeuchte [%] | 2,7 | 3,2 | 2,7 | 2,1 | 2,5 | 2,7 | 2,7 | 2,6 | 2,5 | 2,5 | 2,5 | 2,9 |

**Tabelle VI**

| | | | | | |
|---|---|---|---|---|---|
| Eigenschaften gemäß Beispiel 2 erhaltener pharmazeutischer Präformulierungen, die ein natürliches Gemisch konjugierter equiner Oestrogene als feste, freifließende Trockenextraktfraktion enthalten. | | | | | |

| Versuch Nr. | TS [%] CE [mg/g] CE/TS[%] | g Trockensubstanz/ 1 g Trägerstoff (Vivapor 101) | mg CE Gehalt pro g Trägerstoff | Schüttvolumen [ml/g] | Schüttdichte [g/ml] |
|---|---|---|---|---|---|
| V-A-1 | 3,5 | 277,3 | 85 | 2,36 | 0,42 |
| V-A-2 | 10,726 | | | 2,12 | 0,47 |
| V-A-3 | 30,65 | 293,6 | 90 | 2,16 | 0,46 |
| V-B-1 | 7,4 | 355,6 | 85 | 2,48 | 0,4 |
| V-B-2 | 17,655 | | | 2,5 | 0,4 |
| V-B-3 | 23,9 | | | 2,68 | 0,37 |
| V-C-1 | 12,7 | 322,8 | 85 | 2,84 | 0,35 |
| | 33,451 | | | | |
| V-C-2 | 26,34 | 322,7 | | 2,44 | 0,41 |
| V-D-1 | 14,5 | 496,7 | 90 | 2,28 | 0,44 |
| V-D-2 | 26,274 | | | | |
| | 18,12 | | | 2,12 | 0,47 |
| V-E | 6,6 | 347,5 | 90 | 2,28 | 0,44 |
| | 16,229 | | | | |
| | 24,59 | | | | |
| V-F | 13,6 | 320,2 | 90 | 2,4 | 0,42 |
| | 38,224 | | | | |
| | 28,11 | | | | |

**Tabelle VII**

| **Siebanalysen gemäß Beispiel 2 erhaltener pharmazeutischer Präformulierungen in Form fester, freifließender Trockenextrakte.** | | | | | | | | | | | | | | |
|---|---|---|---|---|---|---|---|---|---|---|---|---|---|---|
| | V-A-1 | | V-B-1 | | V-C-1 | | V-E | | V-F | | Avicel PH 102 | | Vivapur 101 | |
| | V-A-2 | | V-B-2 | | V-C-2 | | | | | | | | | |
| | V-A-3 | | V-B-3 | | | | | | | | | | | |
| **Maschenweite (µm)** | **DG (%)** | **SR (%)** | **DG(%)** | **SR (%)** | **DG (%)** | **SR(%)** | **DG (%)** | **SR (%)** | **DG (%)** | **SR (%)** | **DG (%)** | **SR (%)** | **DG (%)** | **SR (%)** |
| 1000 | 100 | 0 | 100 | 0 | 100 | 0 | 100 | 0 | 100 | 0 | 100 | 0 | 100 | 0 |
| | 100 | 0 | 100 | 0 | 100 | 0 | | | | | | | | |
| | 100 | 0 | 100 | 0 | | | | | | | | | | |
| 500 | 100 | 0 | 100 | 0 | | | 100 | 0 | 100 | 0 | 100 | 0 | 100 | 0 |
| | 100 | 0 | 100 | 0 | 100 | 0 | | | | | | | | |
| | 100 | 0 | 100 | 0 | 100 | 0 | | | | | | | | |
| 250 | 99,59 | 0,41 | 99,17 | 0,83 | 99,20 | 0,80 | 99,39 | 0,61 | 98,33 | 1,67 | 99,48 | 0,52 | 100 | 0 |
| | 99,75 | 0,25 | 99,17 | 0,83 | 99,81 | 0,19 | | | | | | | | |
| | 99,85 | 0,15 | 98,33 | 1,67 | | | | | | | | | | |
| 160 | 92,88 | 6,71 | 88,88 | 10,28 | 81,55 | 17,66 | 83,09 | 16,30 | 86,30 | 12,02 | 88,70 | 10,78 | 100 | 0 |
| | 96,44 | 3,31 | 81,00 | 18,17 | 95,66 | 4,15 | | | | | | | | |
| | 95,54 | 4,32 | 67,32 | 31,01 | | | | | | | | | | |
| 125 | 71,77 | 21,11 | 66,31 | 22,58 | 57,14 | 24,40 | 47,53 | 35,56 | 70,83 | 15,47 | 76,84 | 11,87 | 99,65 | 0,35 |
| | 86,71 | 9,73 | 54,26 | 26,74 | 86,78 | 8,88 | | | | | | | | |
| | 78,70 | 16,84 | 37,94 | 29,98 | | | | | | | | | | |
| 63 | 9,82 | 61,95 | 12,68 | 53,62 | 8,85 | 48,29 | 6,72 | 40,80 | 15,37 | 55,45 | 43,73 | 33,11 | 59,96 | 39,69 |
| | 21,06 | 65,65 | 6,57 | 47,69 | 22,53 | 64,25 | | | | | | | | |
| | 13,97 | 64,73 | 3,93 | 33,42 | | | | | | | | | | |
| Feinanteil | | 9,82 | | 12,68 | | 8,85 | | 6,72 | | 15,37 | | 43,73 | | 59,96 |
| | | 21,06 | | 6,57 | | 22,53 | | | | | | | | |
| | | 13,97 | | 3,93 | | | | | | | | | | |

| | | | | | | | | | | | | | | |
|---|---|---|---|---|---|---|---|---|---|---|---|---|---|---|
| 1) DG = Durchgangssumme / 2) SR = Siebrückstand | | | | | | | | | | | | | | |

### Beispiel 3:

### Orientierende Versuche zur Tablettierung

Um die galenische Weiterverarbeitbarkeit der im Beispiel 1 durch Wirbelschichttechnologie hergestellten Trockenextrakte bzw. Präformulierungen zu testen, wurden die Trockenextrakte bzw. Präformulierungen mit weiteren Tablettierungshilfsstoffen vermischt und zu Matrixtabletten verpreßt. Es zeigte sich, dass die Mischungen in eine Matrixtablette homogen verteilt und verpreßt werden konnten. Überraschenderweise zeigte sich hierbei, daß durch die Wahl des Trägerstoffes und der weiteren Tablettierhilfsstoffe in Abhängigkeit der Wasserlöslichkeit des Träger- und Tablettierhilfsstoff-Gemisches die Freisetzungsgeschwindigkeit der in den Matrixtabletten verpreßten konjugierten Hormone entscheidend beeinflußt werden kann, und dass so erwünschte, vorgegebene Freisetzungsprofile eingestellt werden können. Auch die Zusammensetzung des für die konjugierten Oestrogene als Träger verwendeten Trägerstoffes, z.B. des Gemisches von mikrokristalliner Cellulose mit Lactose, die Partikelgröße und die Porosität des Wirkstoffgranulates, als auch die Partikelgrößenverteilung beeinflussen hierbei die Qualität der Verpreßbarkeit und das Freisetzungsprofil der Hormone, die aus der Matrix freigesetzt werden.

## Patentansprüche

1. Pharmazeutische Präformulierung für die Tablettierung, **gekennzeichnet**
(a) **durch** einen Wirkstoffgehalt eines Gemisches natürlicher konjugierter equiner Oestrogene aus dem Harn trächtiger Stuten, wobei der Wirkstoffgehalt
(i) pro Menge eines pharmazeutischen Trägerstoffes definiert ist, und
(ii) bezogen auf die Haupthormon-Komponenten umfassend Estron, Equilin, 17-α-Dihydroequilin, und gegebenenfalls 17-α-Estradiol und 17-β-Dihydroequilin, jeweils in ihren freien und konjugierten Formen, standardisiert ist;
(b) **dadurch** dass der Wirkstoffgehalt durch Aufsprühen aus einer das Gemisch natürlicher konjugierter equiner Oestrogene enthaltenden wässrigen Lösung auf den pulver- und/oder granulatförmigen pharmazeutischen Trägerstoff aus der Gruppe mikrokristalliner Cellulosen oder einem Gemisch von mikrokristalliner Cellulose mit Lactose und Trocknung aufgebracht ist; und
(c) **dadurch** dass die pharmazeutische Präformulierung in Form eines festen, frei fließenden Trockenextraktes vorliegt.

2. Präformulierung nach Anspruch 1, **dadurch gekennzeichnet, dass** der Wirkstoffgehalt
(a) als Trockensubstanz (TS) eines das Gemisch natürlicher konjugierter equiner Oestrogene enthaltenden Extraktes aus dem Harn trächtiger Stuten (Gesamthormongehalt einschließlich der freien Oestrogene und sonstiger Feststoffe) bezogen auf die Menge des pharmazeutischen Trägerstoffes in der Präformulierung berechnet wird und im Bereich von 0,25 bis 0,70 g TS/g Trägerstoff, vorzugsweise im Bereich von 0,28 bis 0,64 g TS/g Trägerstoff, liegt; oder
(b) als Gemisch natürlicher equiner konjugierter Oestrogene (CE) bezogen auf die Menge des pharmazeutischen Trägerstoffes in der Präformulierung berechnet wird, und im Bereich von 35 bis 100 mg CE/g Trägerstoff, vorzugsweise im Bereich von 43 bis 90 mg CE/g Trägerstoff, liegt; oder
(c) als Gesamthormongehalt (Summe aller konjugierten und freien Hormone) berechnet wird und im Bereich von etwa 35 bis 100 mg pro 1 g des pharmazeutischen Trägerstoffes, vorzugsweise im Bereich von etwa 43 bis 90 mg pro 1 g des pharmazeutischen Trägerstoffes, liegt

3. Pharmazeutische Präformulierung nach Anspruch 2, **dadurch gekennzeichnet, dass** die Restfeuchte in der Präformulierung maximal 3,0 Gew.-%, vorzugsweise maximal 1,0 Gew.-%, bezogen auf die gesamte Präformulierung als 100 Gew.-%, beträgt.

4. Pharmazeutische Präformulierung nach Anspruch 1, **dadurch gekennzeichnet, dass** der auf die Haupthormon-Komponenten standardisierte Wirkstoffgehalt die konjugierten Hormone, in folgenden Anteilen enthält: 52,5 bis 61,5 % Natrium Estronsulfat, 22,5 bis 30,5 % NatriumEquilinsulfat, 13,5 bis 19,5 % Natrium 17-α-Dihydroequilinsulfat, 2,5 bis 9,5 % Natrium Estradiolsulfat, und 0,5 bis 4,0 % Natrium 17-β-Dihydroequilinsulfat.

5. Pharmazeutische Präformulierung nach einem der Ansprüche 1 bis 4, **dadurch gekennzeichnet, dass** der Anteil an freien Hormonen bezogen auf den Gesamtgehalt an Hormonen (Summe aller konjugierten und freien Hormone) im Wirkstoff der Präformulierung, unter 5 Gew.-%, vorzugsweise unter 2 Gew.-%, liegt.

6. Pharmazeutische Präformulierung nach Anspruch 1, **dadurch gekennzeichnet, dass** der pharmazeutische Trägerstoff mikrokristalline Cellulose oder ein Gemisch von mikrokristallinen Cellulosen ist.

7. Pharmazeutische Präformulierung nach Anspruch 1, **dadurch gekennzeichnet, dass** der pharmazeutische Trägerstoff ein Gemisch von mikrokristalliner Cellulose mit Lactose ist.

8. Pharmazeutische Präformulierung nach Anspruch 7, **dadurch gekennzeichnet, dass** das Gewichtsverhältnis von mikrokristalliner Cellulose zu Lactose im Bereich von 8:2 bis 6:4, vorzugsweise im Bereich von etwa 7,5:2,5 bis 6,5:3,5, liegt und insbesondere etwa 7:3 beträgt.

9. Pharmazeutische Präformulierung nach einem der vorhergehenden Ansprüche, **dadurch gekennzeichnet, dass** das mittlere Schüttvolumen der Präformulierung im Bereich von 1,8 bis 3,0 ml/g liegt.

10. Präformulierung nach einem der vorhergehenden Ansprüche, **dadurch gekennzeichnet, dass** das mittlere Schüttgewicht (Schüttdichte) der Präformulierung im Bereich von 0,3 bis 0,6 g/ml liegt.

11. Pharmazeutische Präformulierung nach einem der vorhergehenden Ansprüche, **dadurch gekennzeichnet, dass** die Präformulierung eine durch Siebanalyse als prozentuale Durchgangssumme in Abhängigkeit von der Siebmaschenweite charakterisierte Partikelgrößenverteilung von 100 Gew.-% der Partikel bei einer Maschenweite von 500 µm, von wenigstens 98 Gew.-% der Partikel bei einer Maschenweite von 250 µm, von etwa 65 bis 99,5 Gew.-% der Partikel bei einer Maschenweite von 160 µm, von etwa 35 bis 87 Gew.-% der Partikel bei einer Maschenweite von 125 µm, und einem Feinanteil von unter 23 Gew.-% bei einer Maschenweite von 63 µm, jeweils bezogen auf die Gesamtsumme der Siebfraktionen als 100 Gew.-%, aufweist.

12. Pharmazeutische Präformulierung nach einem der vorhergehenden Ansprüche, **dadurch gekennzeichnet, dass** die Präformulierung eine durch Siebanalyse in Abhängigkeit von der Siebmaschenweite charakterisierte Partikelgrößenverteilung von etwa 0,15 bis maximal 2 Gew.-% der Partikel größer als eine Maschenweite von 250 µm, von etwa 3 bis 31 Gew.-% der Partikel größer als eine Maschenweite von 160 µm, von etwa 8 bis 36 Gew.-% der Partikel größer als eine Maschenweite von 125 µm, und einem Feinanteil der Partikel von etwa 3 bis maximal 23 Gew.-% bei einer Maschenweite von 63 µm, jeweils bezogen auf die Gesamtsumme der Siebfraktionen als 100 Gew.-%, aufweist.

13. Präformulierung nach einem der vorhergehenden Ansprüche, **dadurch gekennzeichnet, dass** die mittlere (durchschnittliche) Partikelgröße der Präformulierung im Bereich von 50 bis 250 µm, vorzugsweise im Bereich von 75 bis 150 µm, liegt.

14. Verfahren zur Herstellung einer pharmazeutischen Präformulierung für die Tablettierung gemäß einem der Ansprüche 1 bis 13, **dadurch gekennzeichnet, dass** man
(a) einen pulver- und/oder granulatförmigen pharmazeutischen Trägerstoff, der aus der Gruppe mikrokristalliner Cellulosen oder einem Gemisch von mikrokristalliner Cellulose mit Lactose ausgewählt ist, in einem Wirbelschichtgerät fluidisiert,
(b) eine wässrige Lösung, die ein Gemisch natürlicher konjugierter equiner Oestrogene aus dem Harn trächtiger Stuten als Wirkstoff enthält, auf den fluidisierten pharmazeutischen Trägerstoff aufsprüht, wobei die wässrige Lösung in einer Menge aufgesprüht wird, die dem in der pharmazeutischen Präformulierung erwünschten, pro Menge des pharmazeutischen Trägerstoffes definierten, und bezogen auf die Haupthormon-Komponenten umfassend Estron, Equilin, 17-α-Dihydroequilin, und gegebenenfalls 17-α-Estradiol und 17-β-Dihydroequilin, jeweils in ihren freien und konjugierten Formen, standardisierten Wirkstoffgehalt entspricht,
(c) die erhaltenen wirkstoffhaltigen Partikel trocknet, und
(d) eine pharmazeutische Präformulierung in Form eines festen, frei fließenden Trockenextraktes erhält.

15. Verfahren nach Anspruch 14, **dadurch gekennzeichnet, dass** die eingesetzte Wirkstoff enthaltende wässrige Lösung neben Wasser als zusätzliches Lösungsmittel noch ein oder mehrere mit Wasser mischbare organische Lösungsmittel, vorzugsweise niedere Alkohole, enthält.

16. Verfahren nach Anspruch 15, **dadurch gekennzeichnet, dass** die eingesetzte Wirkstoff enthaltende wässrige Lösung eine weitgehend rein wässrige Lösung des Gemisches natürlicher konjugierter equiner Oestrogene ist.

17. Verfahren nach einem der Ansprüche 14 bis 16, **dadurch gekennzeichnet, dass** die eingesetzte wässrige Lösung einen Wirkstoffgehalt aufweist, der
(a) als Trockensubstanz des Gemisches natürlicher equiner konjugierter Oestrogene (Gesamthormongehalt einschließlich der freien Oestrogene und sonstiger Feststoffe) berechnet wird und im Bereich von etwa 3,5 bis 20 Gew.-% bezogen auf die wässrige Lösung als 100 Gew.-% liegt, oder
(b) als Gesamthormongehalt (einschließlich der freien Oestrogene) berechnet wird und im Bereich von 10 bis 40 mg pro 1 g der wässrigen Lösung liegt.

18. Verfahren nach einem der Ansprüche 14 bis 16, **dadurch gekennzeichnet, dass** die eingesetzte wässrige Lösung ein Konzentrat ist, das einen Wirkstoffgehalt aufweist, der
(a) als Trockensubstanz des Gemisches natürlicher konjugierter equiner Oestrogene (Gesamthormongehalt einschließlich der freien Oestrogene und sonstiger Feststoffe) berechnet wird und größer als 20 Gew.-% bezogen auf das Konzentrat als 100 Gew.-% ist, oder
(b) als Gemisch natürlicher equiner konjugierter Oestrogene (CE) berechnet wird und bezogen auf die Menge des Konzentrates größer als 40 mg pro 1 g ist.

19. Verfahren nach einem der Ansprüche 14 bis 18, **dadurch gekennzeichnet, dass** der Gesamthormongehalt (einschließlich der freien Oestrogene) in der eingesetzten wässrigen Lösung bezogen auf die darin enthaltene Trockensubstanz als 100 Gew.-% im Bereich von 18 bis 31 Gew.-%, liegt.

20. Verfahren nach einem der Ansprüche 14 bis 18, **dadurch gekennzeichnet, dass** die anhand der Ablufttemperatur regulierte Temperatur des im Wirbelschichtgerät fluidisierten Präformulierungs-Produktes, im Bereich von 25 bis 60 °C, vorzugsweise im Bereich von 45 bis 55 °C, liegt.

21. Verfahren nach einem der Ansprüche 14 bis 18, **dadurch gekennzeichnet, dass** die über die relative Abluftfeuchte regulierte Prozeßfeuchtigkeit im Wirbelschichtgerät, im Bereich von 50 bis 80 % relativer Feuchte liegt.

22. Verfahren nach einem der Ansprüche 14 bis 18, **dadurch gekennzeichnet, dass** die eingesetzte Wirkstoff enthaltende wässrige Lösung mit einer Sprühgeschwindigkeit von 20 bis 50 g/min, auf den pharmazeutischen Trägerstoff aufgesprüht wird.

23. Verfahren nach einem der Ansprüche 14 bis 22, **dadurch gekennzeichnet, dass** der eingesetzte pulver- und/oder granulatförmige pharmazeutische Trägerstoff, insbesondere die mikrokristalline Cellulose, eine durch Siebanalyse als prozentuale Durchgangssumme in Abhängigkeit von der Siebmaschenweite charakterisierte Partikelgrößenverteilung von 100 Gew.-% der Partikel bei einer Maschenweite von 500 µm, von wenigstens 99 Gew.-% der Partikel bei einer Maschenweite von 250 µm, von etwa 85 bis 95 Gew.-% der Partikel bei einer Maschenweite von 160 µm, von etwa 70 bis 80 Gew.-% der Partikel bei einer Maschenweite von 125 µm, und einem Feinanteil von bis zu etwa 50 Gew.-% bei einer Maschenweite von 63 µm, jeweils bezogen auf die Gesamtsumme der Siebfraktionen als 100 Gew.-%, aufweist.

24. Verfahren nach einem der Ansprüche 14 bis 22, **dadurch gekennzeichnet, dass** der eingesetzte pulver- und/oder granulatförmige pharmazeutische Trägerstoff, insbesondere die mikrokristalline Cellulose, eine mittlere (durchschnittliche) Partikelgröße im Bereich von 50 bis 130 µm aufweist.

25. Verfahren nach einem der Ansprüche 14 bis 22, **dadurch gekennzeichnet, dass** der eingesetzte pulver- und/oder granulatförmige pharmazeutische Trägerstoff, insbesondere die mikrokristalline Cellulose, ein Schüttgewicht (Schüttdichte) im Bereich von etwa 25 bis 35 g/ml aufweist.

26. Verfahren nach einem der Ansprüche 14 bis 22, **dadurch gekennzeichnet, dass** der eingesetzte pulver- und/oder granulatförmige pharmazeutische Trägerstoff, insbesondere die mikrokristalline Cellulose, einen Wassergehalt (Trocknungsverlust) von maximal etwa 6 Gew.-% aufweist.

## Claims

1. A pharmaceutical preformulation for tabletting, **characterized**
(a) **by** an active substance content of a mixture of natural, conjugated, equine estrogens from the urine of pregnant mares, wherein the active substance content
(i) is defined per amount of a pharmaceutical support material, and
(ii) is standardized, based on the main hormone components, comprising oestrone, equilin, 17-α-dihydroequilin, and optionally 17-α-oestradial and 17-β-dihydroequilin, each in its free and conjugated form;
(b) in that the active substance content is applied by means of spraying from an aqueous solution containing the mixture of natural, conjugated, equine estrogens onto the powdery and/or granular pharmaceutical support material from the group of microcrystalline celluloses, or a mixture of microcrystalline cellulose with lactose and drying; and
(c) in that the pharmaceutical preformulation is present in the form of a solid, free-flowing dry extract.

2. The preformulation according to claim 1, **characterized in that** the active substance content
(a) is calculated as the dry matter (DM) of an extract containing the mixture of natural, conjugated, equine estrogens from the urine of pregnant mares (total hormone content, including the free estrogens and other solids), based on the amount of the pharmaceutical support material in the preformulation, and is in the range of 0.25 to 0.70 g DM/g of support material, preferably in the range of 0.28 to 0.64 g DM/g of support material; or
(b) is calculated as the mixture of natural, equine, conjugated estrogens (CE), based on the amount of the pharmaceutical support material in the preformulation, and is in the range of 35 to 100 mg CE/g of support material, preferably in the range of 43 to 90 mg CE/g of support material; or
(c) is calculated as the total hormone content (sum of all conjugated and free hormones) and is in the range of about 35 to 100 mg per 1 g of the pharmaceutical support material, preferably in the range of about 43 to 90 mg per 1 g of the pharmaceutical support material.

3. The pharmaceutical preformulation according to claim 2, **characterized in that** the residual moisture in the preformulation is a maximum of 3.0% by weight, preferably a maximum of 1.0% by weight, based on the entire preformulation as 100% by weight.

4. The pharmaceutical preformulation according to claim 1, **characterized in that** the active substance content standardized to the main hormone components contains the conjugated hormones in the following proportions: 52.5 to 61.5% sodium oestrone sulfate, 22.5 to 30.5% sodium equilin sulfate, 13.5 to 19.5% sodium 17-α-dihydroequilin sulfate, 2.5 to 9.5% sodium oestradiol sulfate, and 0.5 to 4.0% sodium 17-β-dihydroequilin sulfate.

5. The pharmaceutical preformulation according to one of the claims 1 to 4, **characterized in that** the proportion of free hormones, based on the total content of hormones (sum of all conjugated and free hormones) in the active substance of the preformulation is less than 5% by weight, preferably less than 2% by weight.

6. The pharmaceutical preformulation according to claim 1, **characterized in that** the pharmaceutical support material is microcrystalline cellulose, or a mixture of microcrystalline celluloses.

7. The pharmaceutical preformulation according to claim 1, **characterized in that** the pharmaceutical support material is a mixture of microcrystalline cellulose with lactose.

8. The pharmaceutical preformulation according to claim 7, **characterized in that** the weight ratio of microcrystalline cellulose to lactose is in the range of 8:2 to 6:4, preferably in the range of about 7.5:2.5 to 6.5:3.5, and is in particular 7:3.

9. The pharmaceutical preformulation according to one of the previous claims, **characterized in that** the mean bulk volume of the preformulation is in the range of 1.8 to 3.0 ml/g.

10. The preformulation according to one of the previous claims, **characterized in that** the mean bulk weight (bulk density) of the preformulation is in the range of 0.3 to 0.6 g/ml.

11. The pharmaceutical preformulation according to one of the previous claims, **characterized in that** the preformulation has a particle size distribution **characterized by** sieve analysis as a percentage throughput total as a function of the sieve mesh size of 100% by weight of the particles at a mesh size of 500 µm, of at least 98% by weight of the particles at a mesh size of 250 µm, of about 65 to 99.5% by weight of the particles at a mesh size of 160 µm, of about 35 to 87% by weight of the particles at a mesh size of 125 µm, and fines of less than 23% by weight at a mesh size of 63 µm, each based on the total of sieve fractions as 100% by weight.

12. The pharmaceutical preformulation according to one of the previous claims, **characterized in that** the preformulation has a particle size distribution **characterized by** sieve analysis as a function of the sieve mesh size of about 0.15 to a maximum of 2% by weight of the particles greater than a mesh size of 250 µm, of about 3 to 31% by weight of the particles greater than a mesh size of 160 µm, of about 8 to 36% by weight of the particles greater than a mesh size of 125 µm, and fines of the particles of about 3 to a maximum of 23% by weight at a mesh size of 63 µm, each based on the total of the sieve fractions as 100% by weight.

13. Preformulation according to one of the previous claims, **characterized in that** the mean (average) particle size of the preformulation is in the range of 50 to 250 µm, preferably in the range of 75 to 150 µm.

14. A method for the production of a pharmaceutical preformulation for tabletting according to the claims 1 to 13, **characterized in that**
(a) a powdery and/or granular pharmaceutical support material, selected from the group of microcrystalline celluloses, or a mixture of microcrystalline cellulose with lactose, is fluidized in a fluidized bed apparatus,
(b) an aqueous solution, containing a mixture of natural, conjugated, equine oestrogens from the urine of pregnant mares as the active substance, is sprayed onto the fluidized pharmaceutical support material, wherein the aqueous solution is sprayed on at an amount corresponding to the standardized active substance content desired in the pharmaceutical preformulation, defined per amount of the pharmaceutical support material, and based on the main hormone components, comprising oestrone, equilin, 17-α-dihydroequilin, and optionally 17-α-oestradiol and 17-β-dihydroequilin, each in its free and conjugated forms,
(c) the obtained particles containing the active substance are dried, and
(d) a pharmaceutical preformulation is obtained in the form of a solid, free-flowing dry extract.

15. The method according to claim 14, **characterized in that** the aqueous solution, containing the active substance which is used contains, in addition to water, one or more water-miscible organic solutions, preferably lower alcohols, as an additional solvent.

16. The method according to claim 15, **characterized in that** the aqueous solution, containing the active substance which is used, is a substantially pure aqueous solution of the mixture of natural, conjugated, equine oestrogens.

17. The method according to claims 14 to 16, **characterized in that** the aqueous solution having an active substance content, which is used
(a) is calculated as a dry matter of the mixture of natural, equine, conjugated oestrogens (total hormone content, including the free oestrogens and other solids), and is in the range of about 3.5 to 20% by weight, based on the aqueous solution as 100% by weight, or
(b) is calculated as a total hormone content (including the free oestrogens), and is in the range of 10 to 40 mg per 1 g of the aqueous solution.

18. The method according to one of the claims 14 to 16, **characterized in that** the aqueous solution which is used is a concentration having an active substance content that is
(a) calculated as a dry matter of the mixture of natural, equine, conjugated oestrogens (total hormone content, including the free oestrogens and other solids), and is greater than 20% by weight, based on the concentration as 10% by weight, or
(b) is calculated as natural, equine, conjugated oestrogen (CE), and based on the amount of the concentration is greater than 40 mg per 1 g.

19. The method according to one of the claims 14 to 18, **characterized in that** the total hormone content (including the free oestrogens) in the aqueous solution used, is in the range of 18 to 31% by weight, based on the dry matter contained therein as 100% by weight.

20. The method according to one of the claims 14 to 18, **characterized in that** the temperature of the preformulation product fluidized in the fluidized-bed apparatus, regulated by the exhaust air temperature, is in the range of 25 to 60°C, preferably in the range of 45 to 55°C.

21. The method according to one of the claims 14 to 18, **characterized in that** the process moisture in the fluidized-bed apparatus, regulated via the relative exhaust air moisture, is in the range of 50 to 80% relative moisture.

22. The method according to one of the clams 14 to 18, **characterized in that** the aqueous solution containing the active substance used, is sprayed onto the pharmaceutical support material at a spraying rate of 20 to 50 g/min.

23. The method according to one of the claims 14 to 22, **characterized in that** the powdery and/or granular pharmaceutical support material used, in particular the microcrystalline cellulose, has a particle size distribution **characterised by** sieve analysis as a percentage throughput total as a function of the sieve mesh size of 100% by weight of the particles at a mesh size of 500 µm, of at least 99% by weight of the particles at a mesh size of 250 µm, of about 85 to 95% by weight of the particles at a mesh size of 160 µm, of about 70 to 80% by weight of the particles at a mesh size of 125 µm, and fines of up to about 50% by weight at a mesh size of 63 µm, each based on the total of the sieve fractions as 100% by weight.

24. The method according to one of the claims 14 to 22, **characterized in that** the powdery and/or granular pharmaceutical support material used, in particular the microcrystalline cellulose, has a mean (average) particle size in the range of 50 to 130 µm.

25. The method according to one of the claims 14 to 22, **characterized in that** the powdery and/or granular pharmaceutical support material used, in particular the microcrystalline cellulose, has a bulk weight (bulk density) in the range of about 25 to 35 g/ml.

26. The method according to one of the claims 14 to 22, **characterized in that** the powdery and/or granular pharmaceutical support material used, in particular the microcrystalline cellulose, has a water content (loss on drying) of a maximum of about 6% by weight.

## Revendications

1. Préformulation pharmaceutique pour la fabrication de comprimés, **caractérisée**
(a) **par** une certaine teneur en un principe actif, constitué d'un mélange d'oestrogènes équins conjugués naturels provenant de l'urine de juments gravides, la teneur en principe actif
(i) étant définie par unité de poids d'un support pharmaceutique, et
(ii) étant normalisée par rapport aux composants hormones principales, comprenant l'oestrone, l'équiline, la 17-α-dihydroéquiline, et éventuellement le 17-α-oestradiol et la 17-β-dihydroéquiline, chacun sous ses formes libres et conjuguées,
(b) en ce que le principe actif contenu est appliqué par pulvérisation, à partir d'une solution aqueuse contenant le mélange d'oestrogènes équins conjugués naturels, sur un support pharmaceutique se présentant sous forme d'une poudre et/ou d'un granulé, support choisi dans le groupe des celluloses microcristallines, ou d'un mélange de cellulose microcristalline et de lactose, et séchage ; et
(c) en ce que la préformulation pharmaceutique se présente sous forme d'un extrait sec solide à écoulement libre.

2. Préformulation selon la revendication 1, **caractérisée en ce que** la teneur en principe actif
(a) est calculée en tant que teneur en matière sèche (TES) d'un extrait, contenant le mélange d'oestrogènes équins conjugués naturels, provenant de l'urine de juments gravides (teneur totale en hormones, y compris les oestrogènes libres et les autres constituants solides), par rapport à la quantité du support pharmaceutique dans la préformulation, et est comprise dans la plage de 0,25 à 0,70 g TES/g de support, de préférence dans la plage de 0,28 à 0,64 g TES/g de support, ou
(b) est calculée en tant que mélange d'oestrogènes conjugués équins naturels (CE) par rapport à la quantité du support pharmaceutique dans la préformulation, et est comprise dans la plage de 35 à 100 mg CE/g de support, de préférence dans la plage de 43 à 90 mg CE/g de support ; ou
(c) est calculée en tant que teneur totale en hormones (somme de toutes les hormones conjuguées et libres), et est comprise dans la plage d'environ 35 à 10O mg/g du support pharmaceutique, de préférence dans la plage d'environ 43 à 90 mg/g du support pharmaceutique.

3. Préformulation pharmaceutique selon la revendication 2, **caractérisé en ce que** l'humidité résiduelle dans la préformulation est au maximum de 3,0 % en poids, de préférence au maximum de 1,0 % en poids, par rapport à la préformulation totale, considérée comme égale à 100 % en poids.

4. Préformulation pharmaceutique selon la revendication 1, **caractérisée en ce que** le principe actif contenu, normalisé aux composants hormones principales, contient les hormones conjuguées selon les proportions suivantes : 52,5 à 61,5 % d'oestronesulfate de sodium, 22,5 à 30,5 % d'équilinesulfate de sodium, 13,5 à 19,5 % de 17-α-dihydroéquilinesulfate de sodium, 2,5 à 9,5 % d'oestradiolsulfate de sodium et 0,5 à 4,0 % de 17-β-dihydroéquitinesulfate de sodium.

5. Préformulation pharmaceutique selon l'une des revendications 1 à 4, **caractérisée en ce que** la proportion des hormones libres, rapportée à la teneur totale en hormones (somme de toutes les hormones conjuguées et libres) dans le principe actif de la préformulation est inférieure à 5 % en poids, de préférence inférieure à 2 % en poids.

6. Préformulation pharmaceutique selon la revendication 1, **caractérisée en ce que** le support pharmaceutique est une cellulose microcristalline ou un mélange de celluloses microcristallines.

7. Préformulation pharmaceutique selon la revendication 1, **caractérisée en ce que** le support pharmaceutique est un mélange de cellulose microcristalline et de lactose.

8. Préformulation pharmaceutique selon la revendication 7, **caractérisée en ce que** le rapport en poids de la cellulose microcristalline au lactose est compris dans la plage de 8:2 à 6:4, de préférence dans la plage d'environ 7,5:2,5 à 6,5:3,5, et est en particulier d'environ 7:3.

9. Préformulation pharmaceutique selon l'une des revendications précédentes, **caractérisée en ce que** le volume massique apparent moyen de la préformulation est compris dans la plage de 1,8 à 3,0 ml/g.

10. Préformulation selon l'une des revendications précédentes, **caractérisée en ce que** la masse volumique apparente moyenne de la préformulation est comprise dans la plage de 0,3 à 0,6 g/ml.

11. Préformulation pharmaceutique selon l'une des revendications précédentes, **caractérisée en ce que** la préformulation présente une distribution granulométrique, **caractérisée par** une analyse granulométrique, en tant que pourcentage de passage en fonction de l'ouverture de maille du tamis, correspondant à 100 % en poids des particules pour une ouverture de maille de 500 µm, à au moins 98 % en poids des particules pour une ouverture de maille de 250 µm, à environ 65 à 99.5 % en poids des particules pour une ouverture de maille de 160 µm, à environ 35 à 87 % en poids des particules pour une ouverture de maille de 125 µm, et à une proportion de fines inférieure à 23 % en poids pour une ouverture de maille de 63 µm, dans chaque cas par rapport au total des fractions granulométriques, considéré comme égal à 100 % en poids.

12. Préformulation pharmaceutique selon l'une des revendications précédentes, **caractérisée en ce que** la préformulation présente une distribution granulométrique, **caractérisée par** une analyse granulométrique en fonction de l'ouverture de maille du tamis, correspondant à environ 0,15 à un maximum de 2 % en poids des particules ayant une granulométrie supérieure à une ouverture de maille de 250 µm, à environ 3 à 31 % en poids des particules ayant une granulométrie supérieure à une ouverture de maille de 160 µm, à environ 8 à 36 % en poids des particules ayant une granulométrie supérieure à une ouverture de maille de 125 µm, et à une proportion de fines des particules d'environ 3 à un maximum de 23 % en poids pour une ouverture de maille de 63 µm, dans chaque cas par rapport au total des fractions granulométriques, considéré comme égal à 100 % en poids.

13. Préformulation selon l'une des revendications précédentes, **caractérisée en ce que** la granulométrie moyenne des particules de la préformulation est comprise dans la plage de 50 à 250 µm, de préférence dans la plage de 75 à 150 µm.

14. Procédé de fabrication d'une préformulation pharmaceutique pour la fabrication de comprimés selon l'une des revendications 1 à 13, **caractérisé en ce que**
(a) on fluidise dans un appareil à lit fluidisé un support pharmaceutique sous forme d'une poudre et/ou d'un granulé, qui est choisi dans le groupe consistant en les celluloses microcristallines ou un mélange de cellulose microcristalline et de lactose,
(b) on pulvérise sur le support pharmaceutique fluidisé une solution aqueuse qui contient en tant que principe actif un mélange d'oestrogènes équins conjugués naturels provenant de l'urine de juments gravides, la solution aqueuse étant pulvérisée en une quantité qui correspond à la teneur en principe actif souhaitée dans la préformulation pharmaceutique, définie par unité de poids de support pharmaceutique, et normalisée par rapport aux composants hormones principales comprenant l'oestrone, l'équiline, la 17-α-dihydroéquiline et éventuellement le 17-α-oestradiol et la 17-β-dihydroéquiline, chacun sous ses formes libres et conjuguées,
(c) on sèche les particules obtenues, contenant le principe actif, et
(d) on obtient une préformulation pharmaceutique sous forme d'un extrait sec solide à écoulement libre.

15. Procédé selon la revendication 14, **caractérisé en ce que** la solution aqueuse contenant le principe actif utilisé contient encore, outre de l'eau, et en tant que solvant additionnel, un ou plusieurs solvants organiques miscibles à l'eau, de préférence des alcools inférieurs.

16. Procédé selon la revendication 15, **caractérisé en ce que** la solution aqueuse contenant le principe actif est une solution aqueuse pratiquement pure du mélange d'oestrogènes équins conjugués naturels.

17. Procédé selon l'une des revendications 14 à 16, **caractérisé en ce que** la solution aqueuse utilisée présente une teneur en principe actif
(a) qui est calculée en tant que teneur en extrait sec du mélange d'oestrogènes conjugués équins naturels (teneur totale en hormones, y compris les oestrogènes libres et les autres matières solides), et qui est comprise dans la plage d'environ 3,5 à 20 % en poids par rapport à la solution aqueuse, considérée comme égale à 100 % en poids, ou
(b) qui est calculée en tant que teneur totale en hormones (y compris les oestrogènes libres), et est comprise dans la plage de 10 à 40 mg/g de la solution aqueuse.

18. Procédé selon l'une des revendications 14 à 16, **caractérisé en ce que** la solution aqueuse utilisée présente une teneur en principe actif
(a) qui est calculée en tant que teneur en extrait sec du mélange d'oestrogènes équins conjugués naturels (teneur totale en hormones, y compris les oestrogènes libres et les autres matières solides), et est supérieur à 20 % en poids par rapport au concentré, considérée comme égale à 100 % en poids, ou
(b) qui est calculée en tant que mélange d'oestrogènes conjugués équins naturels (CE), et, par rapport à la quantité du concentré, est supérieure à 40 mg/g.

19. Procédé selon l'une des revendications 14 à 18, **caractérisé en ce que** la teneur totale en hormones (y compris les oestrogènes libres) de la solution aqueuse utilisée est, par rapport à la teneur en extrait sec de cette dernière, considérée comme égale à 100 % en poids, comprise dans la plage de 18 à 31 % en poids.

20. Procédé selon l'une des revendications 14 à 18, **caractérisé en ce que** la température, régulée par la température de l'air d'évacuation, du produit de préformulation fluidisé dans l'appareil à lit fluidisé, est comprise dans la plage de 25 à 60°C, de préférence dans la plage de 45 à 55°C.

21. Procédé selon l'une des revendications 14 à 18, **caractérisé en ce que** l'humidité due au procédé dans l'appareil à lit fluidisé, régulée par l'intermédiaire de l'humidité relative de l'air d'évacuation, est comprise dans la plage de 50 à 80 % en poids d'humidité relative.

22. Procédé selon l'une des revendications 14 à 18, **caractérisé en ce que** la solution aqueuse utilisée, contenant le principe actif, est pulvérisée sur le support pharmaceutique à une vitesse de pulvérisation de 20 à 50 g/min.

23. Procédé selon l'une des revendications 14 à 22, **caractérisé en ce que** le support pharmaceutique utilisé, sous forme d'une poudre et/ou d'un granulé, en particulier la cellulose microcristalline, présente une distribution granulométrique, **caractérisée par** une analyse granulométrique sous forme d'un pourcentage de passage en fonction de l'ouverture de maille du tamis, correspondant à 100 % en poids des particules pour une ouverture de maille de 500 µm, à au moins 99 % en poids des particules pour une ouverture de maille de 250 µm, à environ 85 à 95 % en poids des particules pour une ouverture de mailles de 160 µm, à environ 70 à 80 % en poids des particules pour une ouverture de maille de 125 µm, et à une proportion de fines allant jusqu'à environ 50 % en poids pour une ouverture de maille de 63 µm, dans chaque cas par rapport au total des fractions granulométriques, considéré comme égal à 100 % en poids.

24. Procédé selon l'une des revendications 14 à 22, **caractérisé en ce que** le support pharmaceutique utilisé, sous forme d'une poudre et/ou d'un granulé, en particulier la cellulose microcristalline, présente une granulométrie moyenne comprise dans la plage de 50 à 130 µm.

25. Procédé selon l'une des revendications 14 à 22, **caractérisé en ce que** le support pharmaceutique utilisé, sous forme d'une poudre et/ou d'un granulé, en particulier la cellulose microcristalline, présente une masse volumique apparente comprise dans la plage d'environ 25 à 35 g/ml.

26. Procédé selon l'une des revendications 14 à 22, **caractérisé en ce que** le support pharmaceutique utilisé, sous forme d'une poudre et/ou d'un granulé, en particulier la cellulose microcristalline, présente une teneur en eau (perte à la dessiccation) au maximum d'environ 6 % en poids.
